# EUROPEAN PATENT APPLICATION

(11) **EP 3 926 639 A1**
(43) Date of publication of application: **22.12.2021**
(21) Application number: 21179128.0
(22) Date of filing: 11.06.2021
(51) Int. Cl.: G16H 20/40, G16H 50/20

(54) **MACHINE LEARNING SYSTEM FOR NAVIGATED ORTHOPEDIC SURGERIES**

(30) Priority: 16.06.2020 US 202016902370
(71) Applicant: Globus Medical, Inc., Audubon, PA 19403 (US)
(72) Inventor: KOSTRZEWSKI, Szymon, 1006 LAUSANNE (CH); GEHRIGER, Daniel, 1004 LAUSANNE (CH); JOHNSON, Norbert, NORTH ANDOVER, 01845 (US); KANAGASEGAR, Keerthighaan, NORRISTOWN, 19403 (US)
(74) Representative: Morabito, Sara

(57) **Abstract**

A surgical guidance system is disclosed for computer assisted navigation during surgery. The surgical guidance system is configured to obtain post-operative feedback data provided by distributed networked computers regarding surgical outcomes for a plurality of patients, and train a machine learning model based on the post-operative feedback data. The surgical guidance system is further configured to obtain pre-operative data from one of the distributed network computers characterizing a defined patient, and generate a surgical plan for the defined patient based on processing the pre-operative data through the machine learning model. The surgical plan is provided to a display device for review by a user.

## Description

### FIELD

The present disclosure relates to medical devices and systems, and more particularly, providing navigation information to users and/or surgical robots for orthopedic surgeries.

### BACKGROUND

There are a number of surgical interventions requiring osteotomy, i.e. cutting an anatomical structure such as a bone along a target plane. Total Knee Arthroplasty (TKA) typically involves cutting both the femoral epiphysis and tibial epiphysis to remove the damaged bone and cartilage and allow installation of a knee prosthesis.

Currently in TKA surgeries patient satisfaction rate is typically about 80%. This is low in comparison to some other types of orthopedic surgeries, such as for hip arthroplasty where patient satisfaction is typically about 95%. These satisfaction rates have remained principally unchanged over several decades despite innovations in:
- New implant designs;
- Computer-assisted surgery (CAS): navigation and robotic surgery system;
- Custom cutting template solutions; and
- Customized implants.

This suggests that there are problems with TKA and other orthopedic surgeries that have not been addressed by previous medical procedures and related innovations.

### SUMMARY

Some embodiments of the present disclosure are directed to a surgical guidance system for computer assisted navigation during surgery. The surgical guidance system is configured to obtain post-operative feedback data provided by distributed networked computers regarding surgical outcomes for a plurality of patients, and train a machine learning model based on the post-operative feedback data. The surgical guidance system is further configured to obtain pre-operative data from one of the distributed network computers characterizing a defined patient, and generate a surgical plan for the defined patient based on processing the pre-operative data through the machine learning model. The surgical plan is provided to a display device for review by a user.

Some other embodiments are directed to a surgical system that includes a surgical guidance system, a tracking system, in at least one controller. The surgical guidance system is configured to obtain post-operative feedback data provided by distributed networked computers regarding surgical outcomes for a plurality of patients, and train a machine learning model based on the post-operative feedback data. The surgical guidance system is further configured to obtain pre-operative data from one of the distributed network computers characterizing a defined patient, and generate a surgical plan for the defined patient based on processing the pre-operative data through the machine learning model. The surgical plan is provided to a display device for review by a user. The tracking system is configured to determine a pose of an anatomical structure of the defined patient that is to be cut by a surgical saw and to determine a pose of the surgical saw. The at least one controller is configured to obtain the surgical plan from the surgical guidance system, determine a pose of a target plane based on the surgical plan defining where the anatomical structure is to be cut and based on the pose of the anatomical structure, and generate steering information based on comparison of the pose of the target plane and the pose of the surgical saw, wherein the steering information indicates where the surgical saw needs to be moved to position a cutting plane of the surgical saw to become aligned with the target plane.

Other surgical systems, surgical guidance systems, and corresponding methods and computer program products according to embodiments will be or become apparent to one with skill in the art upon review of the following drawings and detailed description. It is intended that all such surgical systems, surgical guidance systems, and corresponding methods and computer program products be included within this description, be within the scope of the present disclosure, and be protected by the accompanying claims. Moreover, it is intended that all embodiments disclosed herein can be implemented separately or combined in any way and/or combination.

### BRIEF DESCRIPTION OF THE DRAWINGS

The accompanying drawings, which are included to provide a further understanding of the disclosure and are incorporated in a constitute a part of this application, illustrate certain non-limiting embodiments of inventive concepts. In the drawings:
Figure 1 illustrates an embodiment of a surgical system according to some embodiments of the present disclosure;
Figure 2 illustrates a surgical robot component of the surgical system of Figure 1 according to some embodiments of the present disclosure;
Figure 3A illustrates a camera tracking system component of the surgical system of

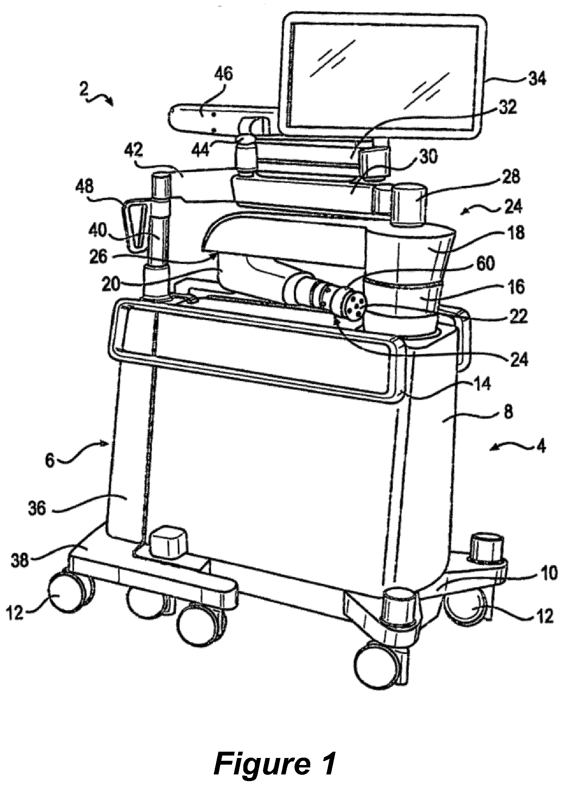

Figure 1 according to some embodiments of the present disclosure;

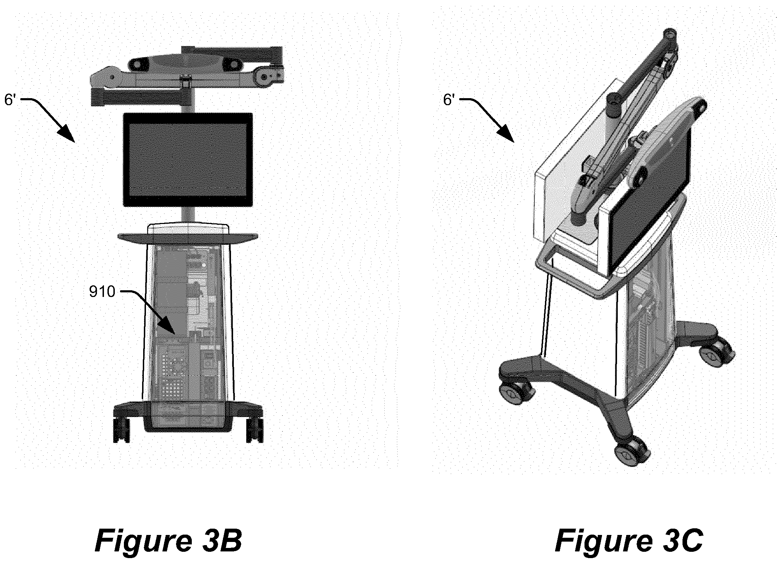

Figures 3B and 3C illustrate a front view and isometric view of another camera tracking system component which may be used with the surgical system of Figure 1 according to some embodiments of the present disclosure;

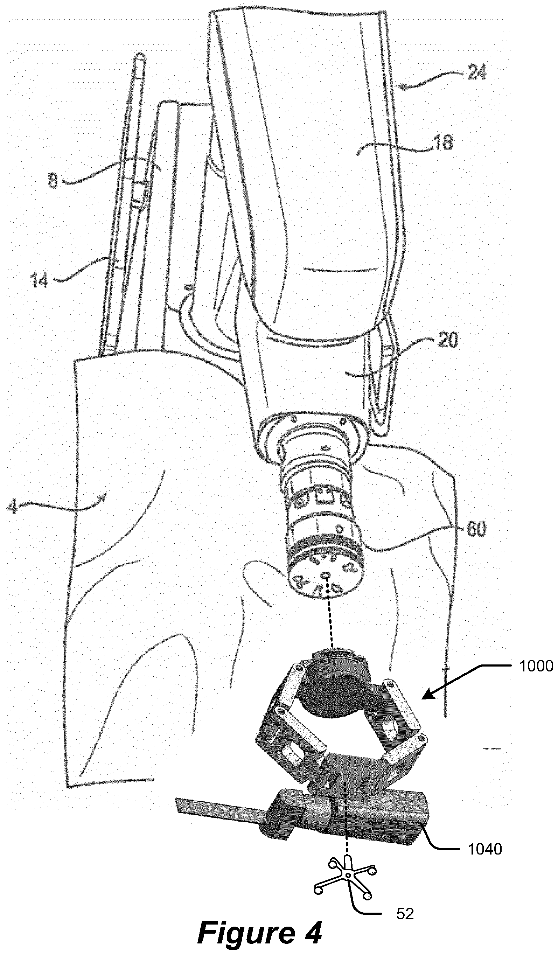

Figure 4 illustrates an embodiment of an end effector that is connectable to a robot arm and configured according to some embodiments of the present disclosure;

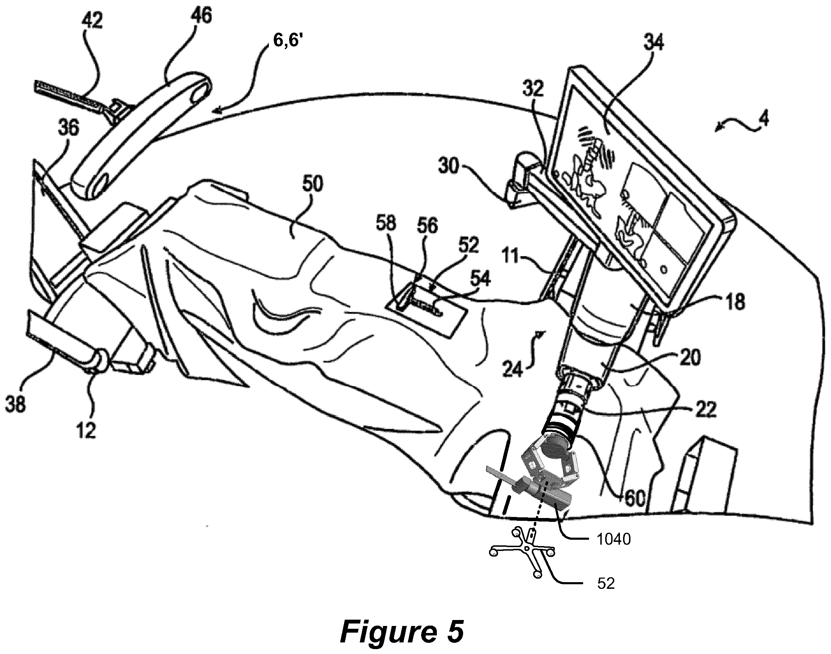

Figure 5 illustrates a medical operation in which a surgical robot and a camera system are disposed around a patient;

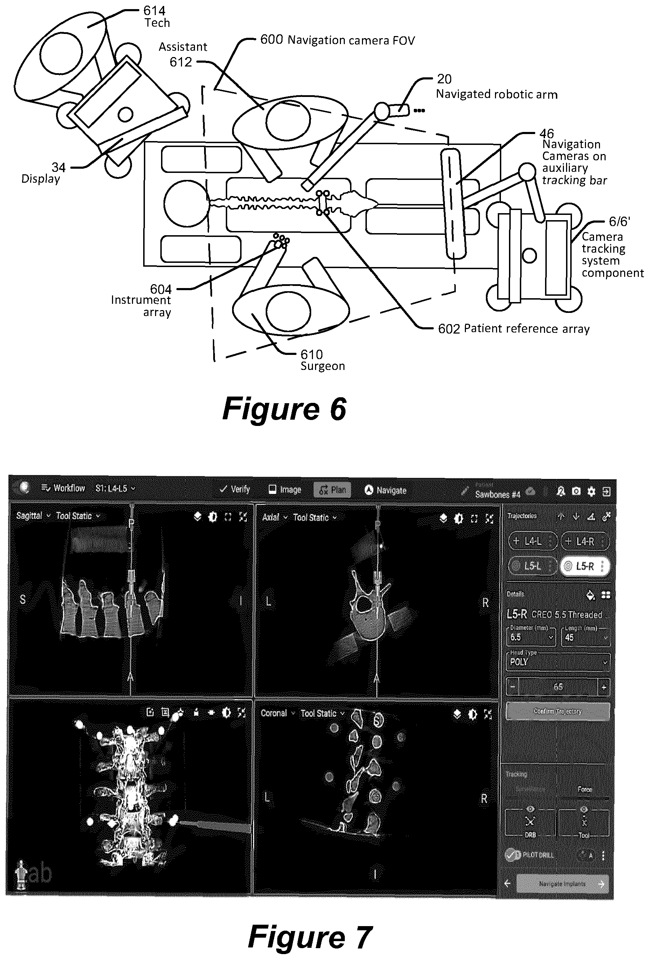

Figure 6 illustrates a block diagram view of the components of the surgical system of
Figure 5 being used for a medical operation;
Figure 7 illustrates various display screens that may be displayed on the display of
Figures 5 and 6 when using a navigation function of the surgical system;

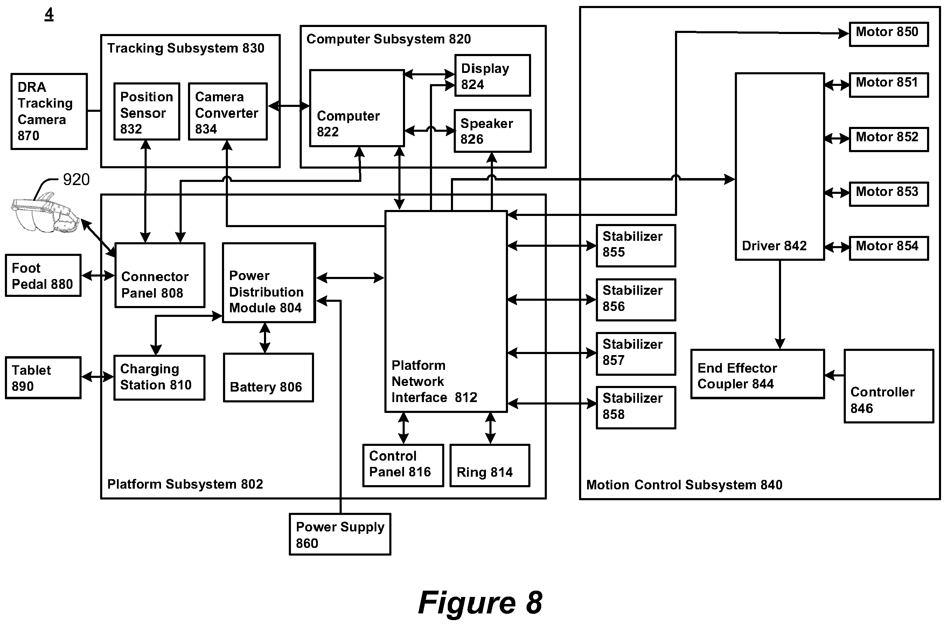

Figure 8 illustrates a block diagram of some electrical components of a surgical robot according to some embodiments of the present disclosure;

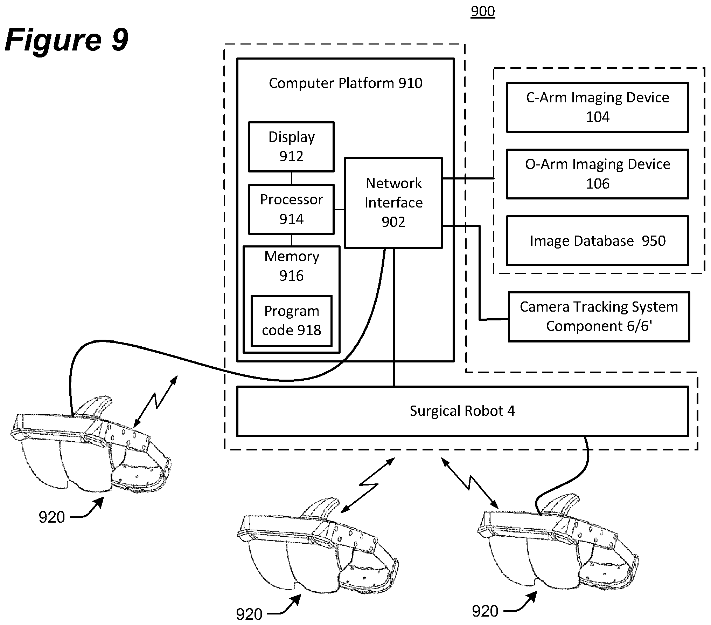

Figure 9 illustrates a block diagram of components of a surgical system that includes imaging devices connected to a computer platform which can be operationally connected to a camera tracking system and/or surgical robot according to some embodiments of the present disclosure;

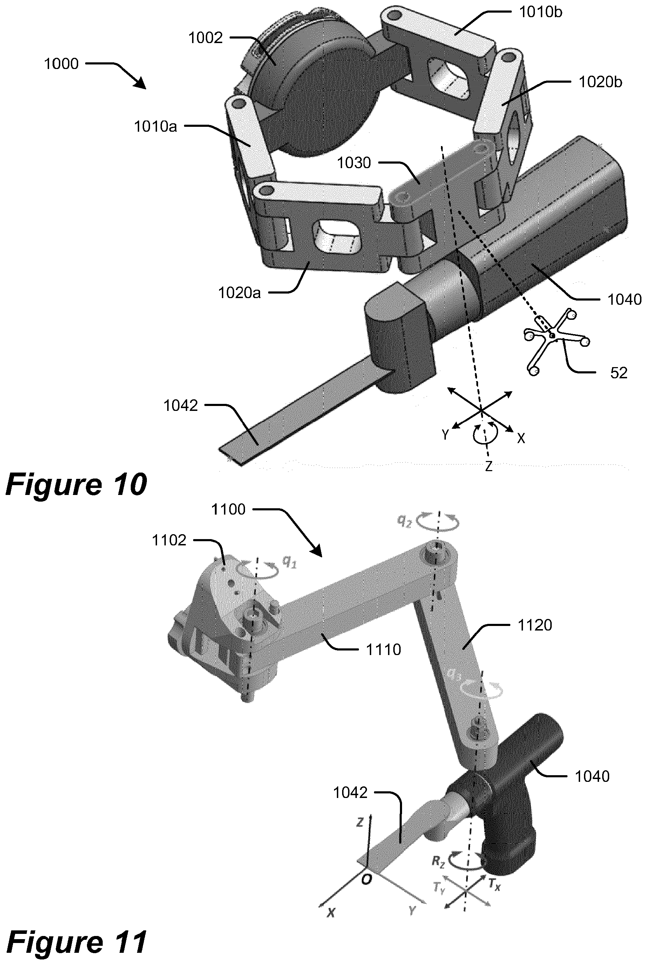

Figures 10 and 11 illustrate alternative embodiments of passive end effectors which are configured in accordance with some embodiments of the present disclosure;

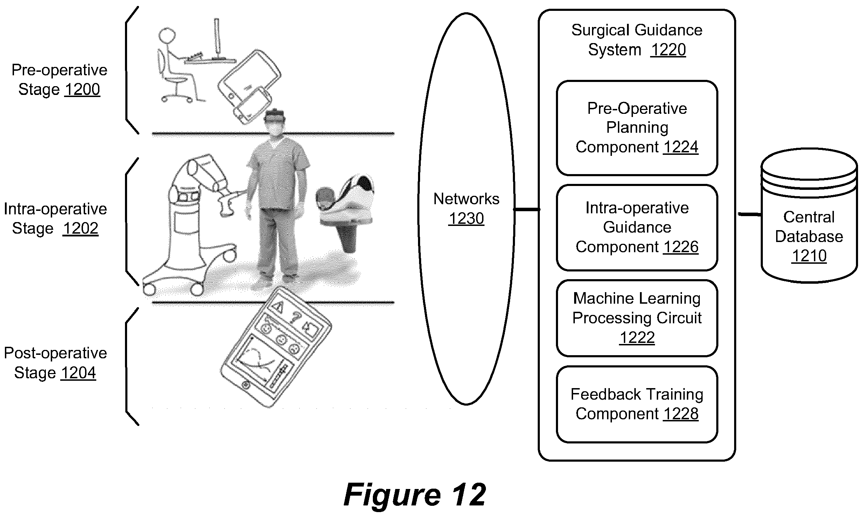

Figure 12 illustrates a navigated surgery workflow which uses a surgical guidance system configured in accordance with some embodiments;

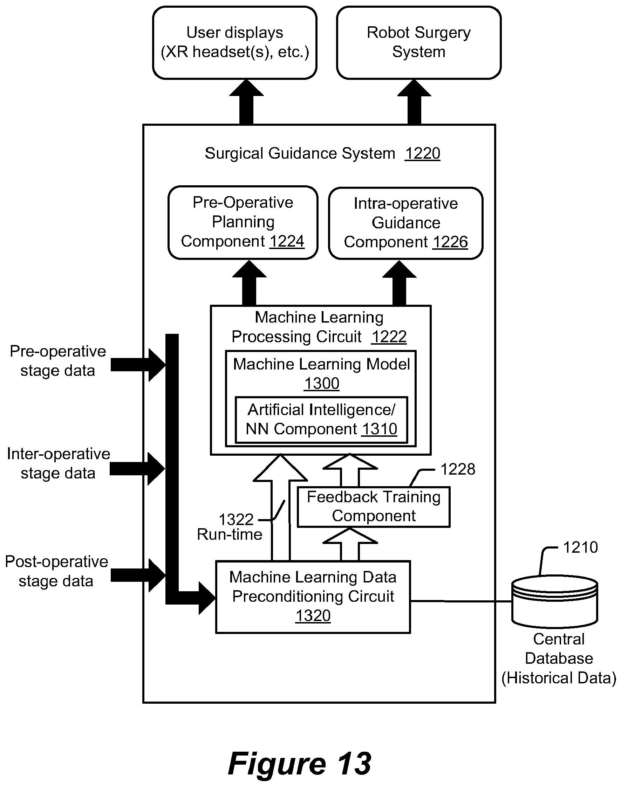

Figure 13 illustrates a block diagram of the surgical guidance system with associated data flows during the pre-operative, intra-operative, and post-operative stages, and shows surgical guidance being provided to user displays and to a robot surgery system in accordance with some embodiments;

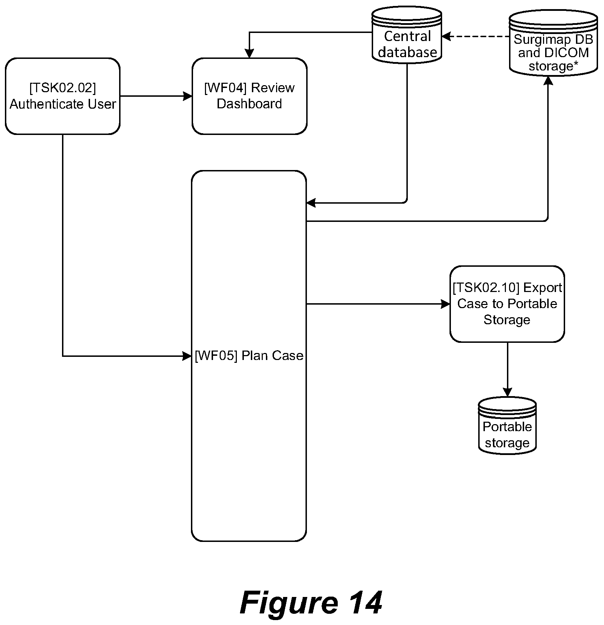

Figure 14 illustrates functional blocks performing a pre-operative plan workflow, and which may be at least partially performed by the surgical guidance system in accordance with some embodiments;

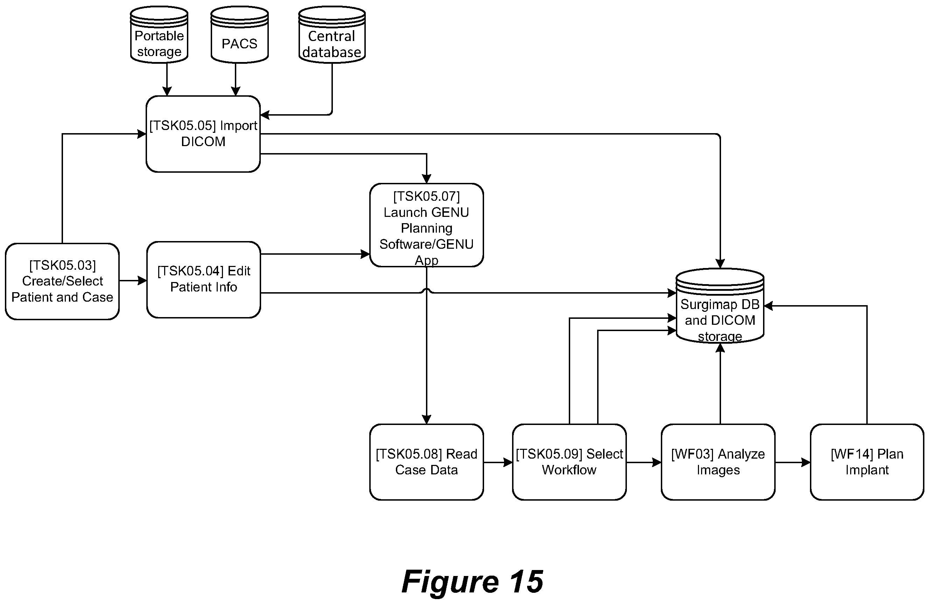

Figure 15 illustrates functional blocks performing an example surgical case plan in accordance with some embodiments;

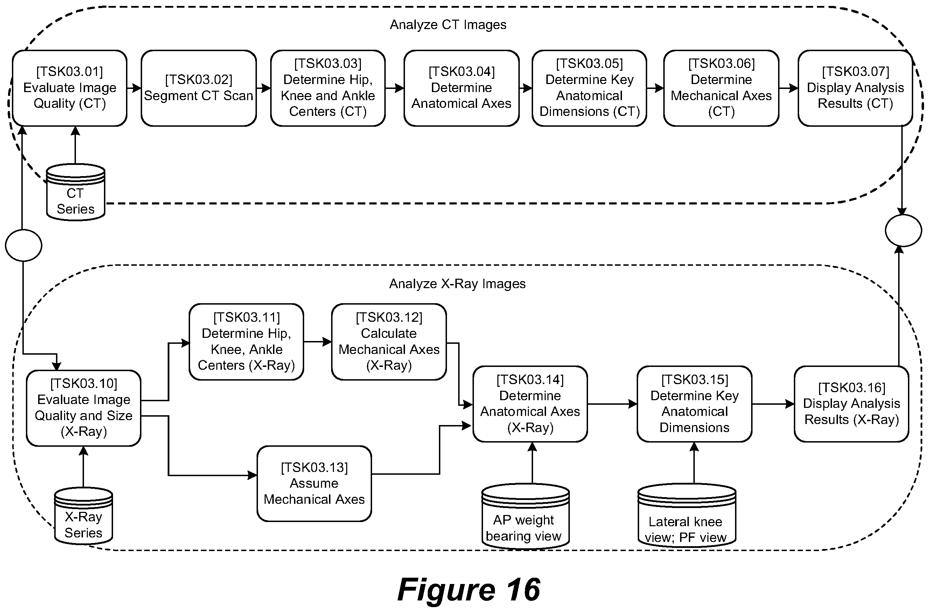

Figure 16 illustrates functional blocks for image analysis and which may be at least partially performed by the surgical guidance system in accordance with some embodiments;

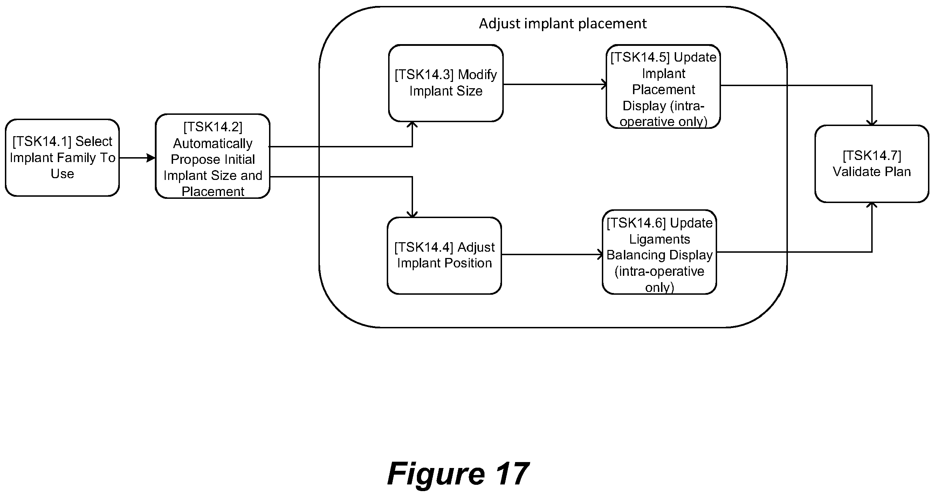

Figure 17 illustrates functional blocks for a plan device implant workflow and which may be at least partially performed by the surgical guidance system in accordance with some embodiments;

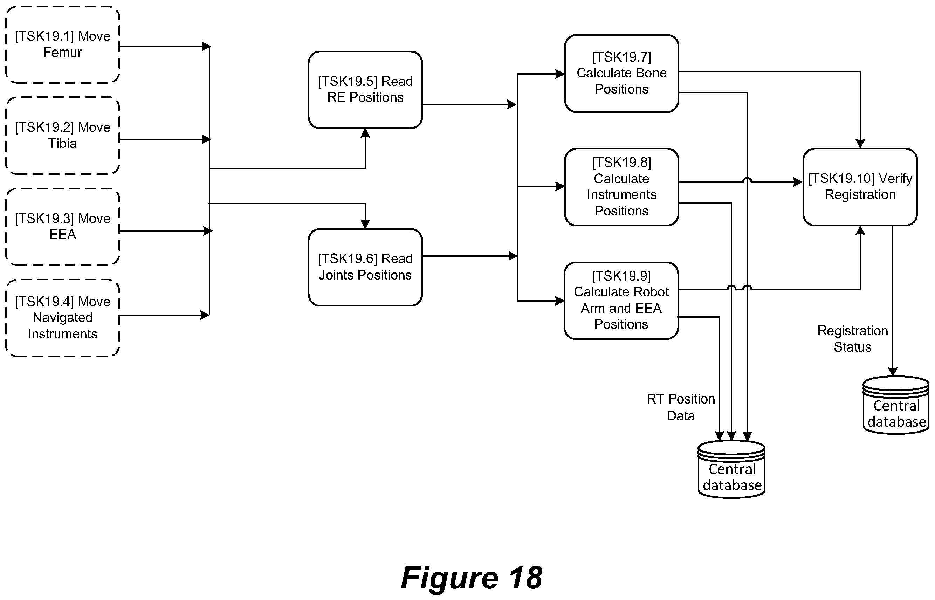

Figure 18 illustrates functional blocks for navigated workflow and which may be at least partially performed by the surgical guidance system, in accordance with some embodiments;

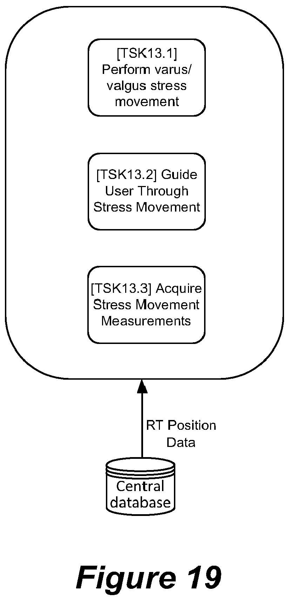

Figure 19 illustrates functional blocks for testing Range of Motion (ROM) workflow and which may be at least partially performed by the surgical guidance system, in accordance with some embodiments;

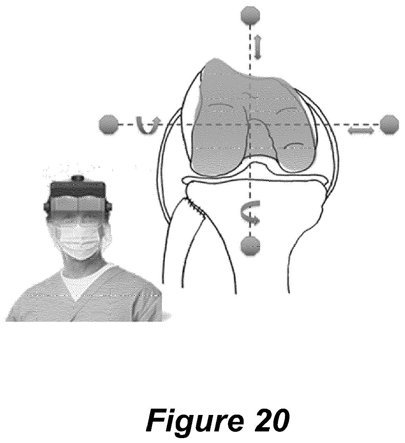

Figure 20 illustrates part of a surgical plan is displayed through an XR headset as an overlay on a patient's bone to assist with implant position planning in accordance with some embodiments;

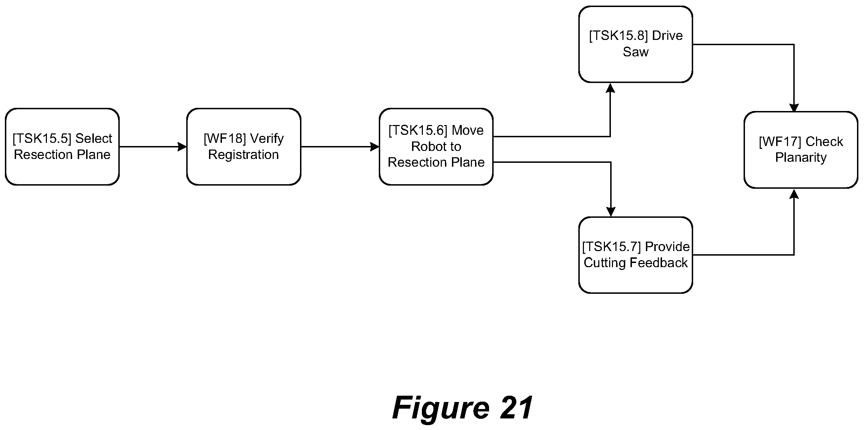

Figure 21 shows a surgical robot workflow performed to make one or more cuts on a bone according to a surgical plan in accordance with some embodiments;

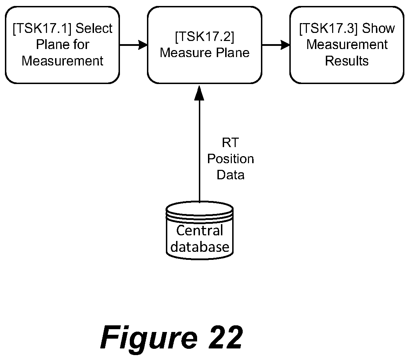

Figure 22 shows a check planarity workflow which may be at least partially performed by the surgical guidance system in accordance with some embodiments;

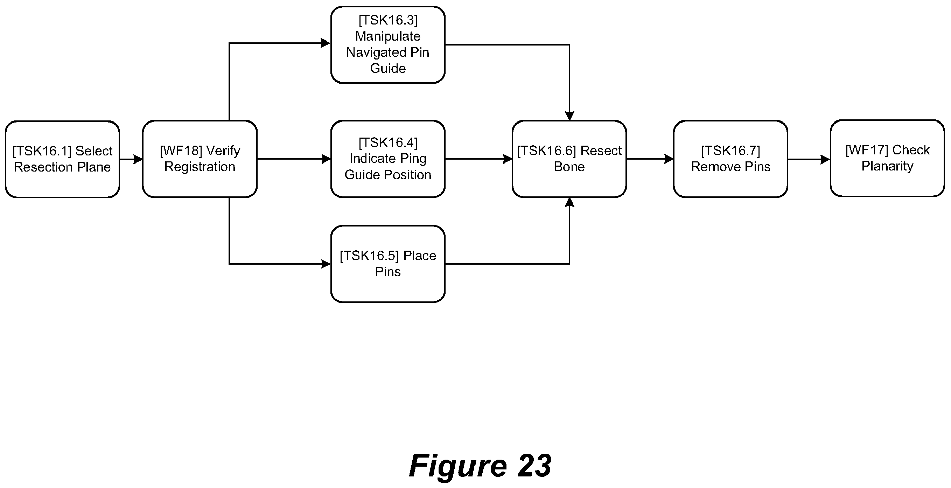

Figure 23 shows a workflow to cut bones with navigated jigs which may be at least partially performed by the surgical guidance system in accordance with some embodiments;

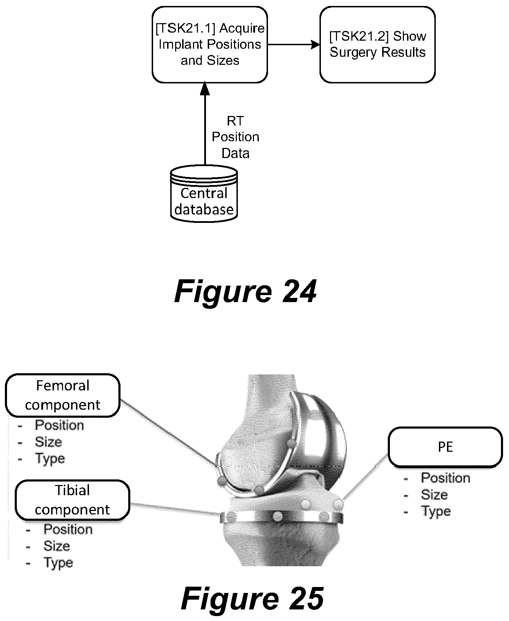

Figure 24 shows a workflow to evaluate results of implantation of the implant device, which may be at least partially performed by the surgical guidance system in accordance with some embodiments;
Figure 25 shows divots provided on implant devices which can have their collective poses tracked by the camera tracking system in accordance with some embodiments; and

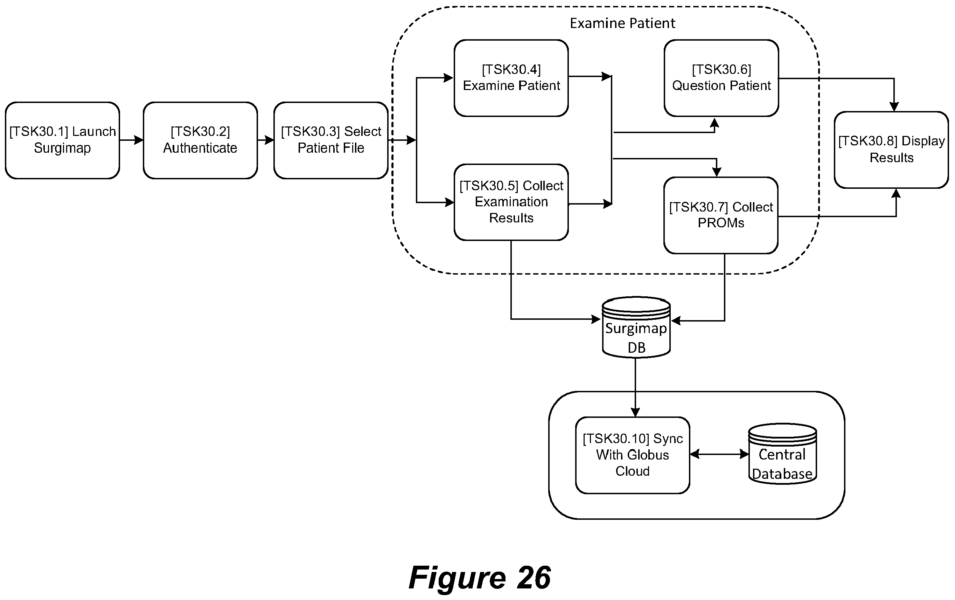

Figure 26 shows a patient examination workflow which may be at least partially performed by the surgical guidance system in accordance with some embodiments.

### DETAILED DESCRIPTION

Inventive concepts will now be described more fully hereinafter with reference to the accompanying drawings, in which examples of embodiments of inventive concepts are shown. Inventive concepts may, however, be embodied in many different forms and should not be construed as limited to the embodiments set forth herein. Rather, these embodiments are provided so that this disclosure will be thorough and complete, and will fully convey the scope of various present inventive concepts to those skilled in the art. It should also be noted that these embodiments are not mutually exclusive. Components from one embodiment may be tacitly assumed to be present or used in another embodiment.

Various embodiments disclosed herein are directed to improvements in operation of a surgical system providing navigated guidance when planning for and performing orthopedic surgical procedures, such as osteotomy for joint implants. A machine learning (ML) guidance system provides patient customized guidance during pre-operative stage planning, intra-operative stage surgical procedures, and post-operative stage assessment. A central database stores data that can be obtained in each of the stages across all patients who have previously used or are currently using the ML guidance system. The ML system is trained over time based on data from the central database so that the patient customized guidance provides improved surgical outcomes.

Figure 1 illustrates an embodiment of a surgical system 2 according to some embodiments of the present disclosure. Prior to performance of an orthopedic or other surgical procedure, a three-dimensional ("3D") image scan may be taken of a planned surgical area of a patient using, e.g., the C-Arm imaging device 104 of Fig. 10 or O-Arm imaging device 106 of Fig. 11, or from another medical imaging device such as a computed tomography (CT) image or MRI. This scan can be taken pre-operatively (e.g. few weeks before procedure, most common) or intra-operatively. However, any known 3D or 2D image scan may be used in accordance with various embodiments of the surgical system 2. The image scan is sent to a computer platform in communication with the surgical system 2, such as the computer platform 910 of the surgical system 900 (Fig. 9) which may include the camera tracking system component 6, the surgical robot 4 (e.g., robot 2 in Fig. 1), imaging devices (e.g., C-Arm 104, O-Arm 106, etc.), and an image database 950 for storing image scans of patients. A surgeon reviewing the image scan(s) on a display device of the computer platform 910 (Fig. 9) generates a surgical plan defining a target pose for a surgical tool to be used during a surgical procedure on an anatomical structure of the patient. Example surgical tools, also referred to as tools, can include, without limitation, drills, screw drivers, saws, retractors, and implants such as a screws, spacers, interbody fusion devices, plates, rods, etc. In some embodiments, the surgical plan defining the target plane is planned on the 3D image scan displayed on a display device.

As used herein, the term "pose" refers to the position and/or the rotational angle of one object (e.g., dynamic reference array, end effector, surgical tool, anatomical structure, etc.) relative to another object and/or to a defined coordinate system. A pose may therefore be defined based on only the multidimensional position of one object relative to another object and/or to a defined coordinate system, only on the multidimensional rotational angles of the object relative to another object and/or to a defined coordinate system, or on a combination of the multidimensional position and the multidimensional rotational angles. The term "pose" therefore is used to refer to position, rotational angle, or combination thereof.

The surgical system 2 of Figure 1 can assist surgeons during medical procedures by, for example, holding tools, aligning tools, using tools, guiding tools, and/or positioning tools for use. In some embodiments, surgical system 2 includes a surgical robot 4 and a camera tracking system component 6. The ability to mechanically couple surgical robot 4 and camera tracking system component 6 can allow for surgical system 2 to maneuver and move as a single unit, and allow surgical system 2 to have a small footprint in an area, allow easier movement through narrow passages and around turns, and allow storage within a smaller area.

An orthopedic surgical procedure may begin with the surgical system 2 moving from medical storage to a medical procedure room. The surgical system 2 may be maneuvered through doorways, halls, and elevators to reach a medical procedure room. Within the room, the surgical system 2 may be physically separated into two separate and distinct systems, the surgical robot 4 and the camera tracking system 6. Surgical robot 4 may be positioned adjacent the patient at any suitable location to properly assist medical personnel. Camera tracking system component 6 may be positioned at the base of the patient, at patient shoulders or any other location suitable to track the present pose and movement of the pose of tracks portions of the surgical robot 4 and the patient. Surgical robot 4 and Camera tracking system component 6 may be powered by an onboard power source and/or plugged into an external wall outlet.

Surgical robot 4 may be used to assist a surgeon by holding and/or using tools during a medical procedure. To properly utilize and hold tools, surgical robot 4 may rely on a plurality of motors, computers, and/or actuators to function properly. Illustrated in Figure 1, robot body 8 may act as the structure in which the plurality of motors, computers, and/or actuators may be secured within surgical robot 4. Robot body 8 may also provide support for robot telescoping support arm 16. The size of robot body 8 may provide a solid platform supporting attached components, and may house, conceal, and protect the plurality of motors, computers, and/or actuators that may operate attached components.

Robot base 10 may act as a lower support for surgical robot 4. In some embodiments, robot base 10 may support robot body 8 and may attach robot body 8 to a plurality of powered wheels 12. This attachment to wheels may allow robot body 8 to move in space efficiently. Robot base 10 may run the length and width of robot body 8. Robot base 10 may be about two inches to about 10 inches tall. Robot base 10 may cover, protect, and support powered wheels 12.

some embodiments, as illustrated in Figure 1, at least one powered wheel 12 may be attached to robot base 10. Powered wheels 12 may attach to robot base 10 at any location. Each individual powered wheel 12 may rotate about a vertical axis in any direction. A motor may be disposed above, within, or adjacent to powered wheel 12. This motor may allow for surgical system 2 to maneuver into any location and stabilize and/or level surgical system 2. A rod, located within or adjacent to powered wheel 12, may be pressed into a surface by the motor. The rod, not pictured, may be made of any suitable metal to lift surgical system 2. The rod may lift powered wheel 10, which may lift surgical system 2, to any height required to level or otherwise fix the orientation of the surgical system 2 in relation to a patient. The weight of surgical system 2, supported through small contact areas by the rod on each wheel, prevents surgical system 2 from moving during a medical procedure. This rigid positioning may prevent objects and/or people from moving surgical system 2 by accident.

Moving surgical system 2 may be facilitated using robot railing 14. Robot railing 14 provides a person with the ability to move surgical system 2 without grasping robot body 8. As illustrated in Figure 1, robot railing 14 may run the length of robot body 8, shorter than robot body 8, and/or may run longer the length of robot body 8. Robot railing 14 may further provide protection to robot body 8, preventing objects and or personnel from touching, hitting, or bumping into robot body 8.

Robot body 8 may provide support for a Selective Compliance Articulated Robot Arm, hereafter referred to as a "SCARA." A SCARA 24 may be beneficial to use within the surgical system 2 due to the repeatability and compactness of the robotic arm. The compactness of a SCARA may provide additional space within a medical procedure, which may allow medical professionals to perform medical procedures free of excess clutter and confining areas. SCARA 24 may comprise robot telescoping support 16, robot support arm 18, and/or robot arm 20. Robot telescoping support 16 may be disposed along robot body 8. As illustrated in Figure 1, robot telescoping support 16 may provide support for the SCARA 24 and display 34. In some embodiments, robot telescoping support 16 may extend and contract in a vertical direction. The body of robot telescoping support 16 may be any width and/or height configured to support the stress and weight placed upon it.

In some embodiments, medical personnel may move SCARA 24 through a command submitted by the medical personnel. The command may originate from input received on display 34, a tablet, and/or an XR headset (e.g., headset 920 in Fig. 9) as will be explained in further detail below. The XR headset may eliminate the need for medical personnel to refer to any other display such as the display 34 or a tablet, which enables the SCARA 24 to be configured without the display 34 and/or the tablet. The command may be generated by the depression of a switch and/or the depression of a plurality of switches, and/or may be generated based on a hand gesture command and/or voice command that is sensed by the XR headset as will be explained in further detail below.

An activation assembly 60 may include a switch and/or a plurality of switches. The activation assembly 60 may be operable to transmit a move command to the SCARA 24 allowing an operator to manually manipulate the SCARA 24. When the switch, or plurality of switches, is depressed the medical personnel may have the ability to move SCARA 24 through applied hand movements. Alternatively or additionally, an operator may control movement of the SCARA 24 through hand gesture commands and/or voice commands that are sensed by the XR headset as will be explained in further detail below. Additionally, when the SCARA 24 is not receiving a command to move, the SCARA 24 may lock in place to prevent accidental movement by personnel and/or other objects. By locking in place, the SCARA 24 provides a solid platform through which the end effector 26 can guide a surgical tool during a medical procedure.

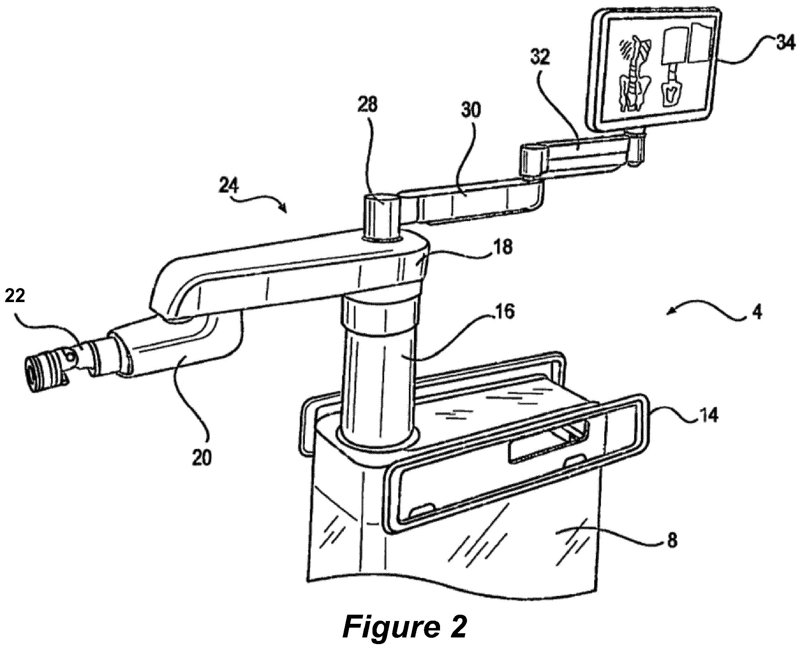

Robot support arm 18 can be connected to robot telescoping support 16 by various mechanisms. In some embodiments, best seen in Figures 1 and 2, robot support arm 18 rotates in any direction in regard to robot telescoping support 16. Robot support arm 18 may rotate three hundred and sixty degrees around robot telescoping support 16. Robot arm 20 may connect to robot support arm 18 at any suitable location and by various mechanisms that enable rotation in any direction relative to robot support arm 18. In one embodiment, the robot arm 20 can rotate three hundred and sixty degrees relative to the robot support arm 18. This free rotation allows an operator to position robot arm 20 according to a surgical plan.

The passive end effector 1000 in Figures 4 and 5 may attach to robot arm 20 in any suitable location. As will be explained in further detail below, the passive end effector 1000 can include a base, a first mechanism, and a second mechanism. The base is configured to attach to an end effector coupler 22 of the robot arm 20 positioned by the surgical robot 4. The first mechanism extends between a rotatable connection to the base and a rotatable connection to a tool attachment mechanism. The second mechanism extends between a rotatable connection to the base and a rotatable connection to the tool attachment mechanism. The first and second mechanisms pivot about the rotatable connections, and may be configured to constrain movement of the tool attachment mechanism to a range of movement within a working plane. The rotatable connections may be pivot joints allowing 1 degree-of-freedom (DOF) motion, universal joints allowing 2 DOF motions, or ball joints allowing 3 DOF motions. The tool attachment mechanism is configured to connect to a surgical saw 1040 having a saw blade. The surgical saw 1040 may be configured to oscillate the saw blade for cutting. The first and second mechanisms may be configured to constrain a cutting plane of the saw blade to be parallel to the working plane. Pivot joints may be preferably used for connecting the planar mechanisms when the passive end effector is to be configured to constrain motion of the saw blade to the cutting plane.

The tool attachment mechanism may connect to the surgical saw 1040 through various mechanisms that can include, but are not limited to, a screw, nut and bolt, clamp, latch, tie, press fit, or magnet. In some embodiments, a dynamic reference array 52 is attached to the passive end effector 1000, e.g., to the tool attachment mechanism, and/or is attached to the surgical saw 1040. Dynamic reference arrays, also referred to as "DRAs" and "reference arrays" herein, can be rigid bodies, markers, or other indicia which may be attached or formed on one or more XR headsets being worn by personnel in the operating room, the end effector, the surgical robot, a surgical tool in a navigated surgical procedure, and an anatomical structure (e.g., bone) of a patient. The computer platform 910 in combination with the camera tracking system component 6 or other 3D localization system are configured to track in real-time the pose (e.g., positions and rotational orientations) of the DRA. The DRA can include fiducials, such as the illustrated arrangement of balls. This tracking of 3D coordinates of the DRA can allow the surgical system 2 to determine the pose of the DRA in any multidimensional space in relation to the target anatomical structure of the patient 50 in Figure 5.

As illustrated in Figure 1, a light indicator 28 may be positioned on top of the SCARA 24. Light indicator 28 may illuminate as any type of light to indicate "conditions" in which surgical system 2 is currently operating. In some embodiments, the light may be produced by LED bulbs, which may form a ring around light indicator 28. Light indicator 28 may comprise a fully permeable material that can let light shine through the entirety of light indicator 28. Light indicator 28 may be attached to lower display support 30. Lower display support 30, as illustrated in Figure 2 may allow an operator to maneuver display 34 to any suitable location. Lower display support 30 may attach to light indicator 28 by any suitable mechanism. In some embodiments, lower display support 30 may rotate about light indicator 28 or be rigidly attached thereto. Upper display support 32 may attach to lower display support 30 by any suitable mechanism.

In some embodiments, a tablet may be used in conjunction with display 34 and/or without display 34. The tablet may be disposed on upper display support 32, in place of display 34, and may be removable from upper display support 32 during a medical operation. In addition the tablet may communicate with display 34. The tablet may be able to connect to surgical robot 4 by any suitable wireless and/or wired connection. In some embodiments, the tablet may be able to program and/or control surgical system 2 during a medical operation. When controlling surgical system 2 with the tablet, all input and output commands may be duplicated on display 34. The use of a tablet may allow an operator to manipulate surgical robot 4 without having to move around patient 50 and/or to surgical robot 4.

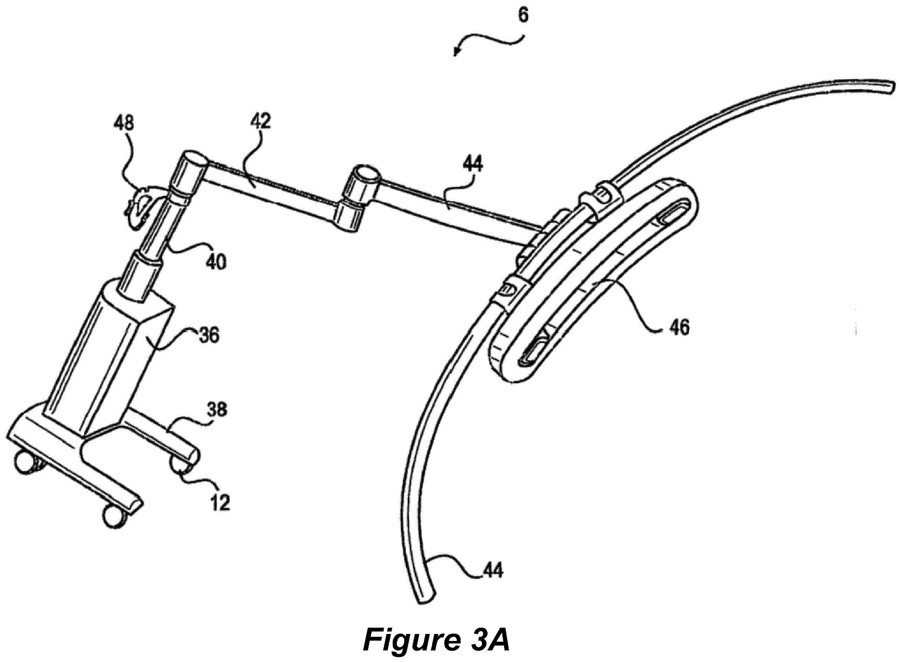

As illustrated in Figures 3A and 5, camera tracking system component 6 works in conjunction with surgical robot 4 through wired or wireless communication networks. Referring to Figures 1, 3 and 5, camera tracking system component 6 can include some similar components to the surgical robot 4. For example, camera body 36 may provide the functionality found in robot body 8. Robot body 8 may provide an auxiliary tracking bar upon which cameras 46 are mounted. The structure within robot body 8 may also provide support for the electronics, communication devices, and power supplies used to operate camera tracking system component 6. Camera body 36 may be made of the same material as robot body 8. Camera tracking system component 6 may communicate directly to an XR headset, tablet and/or display 34 by a wireless and/or wired network to enable the XR headset, tablet and/or display 34 to control the functions of camera tracking system component 6.

Camera body 36 is supported by camera base 38. Camera base 38 may function as robot base 10. In the embodiment of Figure 1, camera base 38 may be wider than robot base 10. The width of camera base 38 may allow for camera tracking system component 6 to connect with surgical robot 4. As illustrated in Figure 1, the width of camera base 38 may be large enough to fit outside robot base 10. When camera tracking system component 6 and surgical robot 4 are connected, the additional width of camera base 38 may allow surgical system 2 additional maneuverability and support for surgical system 2.

As with robot base 10, a plurality of powered wheels 12 may attach to camera base 38. Powered wheel 12 may allow camera tracking system component 6 to stabilize and level or set fixed orientation in regards to patient 50, similar to the operation of robot base 10 and powered wheels 12. This stabilization may prevent camera tracking system component 6 from moving during a medical procedure and may keep cameras 46 on the auxiliary tracking bar from losing track of a DRA connected to an XR headset and/or the surgical robot 4, and/or losing track of one or more DRAs 52 connected to an anatomical structure 54 and/or tool 58 within a designated area 56 as shown in Figures 3A and 5. This stability and maintenance of tracking enhances the ability of surgical robot 4 to operate effectively with camera tracking system component 6. Additionally, the wide camera base 38 may provide additional support to camera tracking system component 6. Specifically, a wide camera base 38 may prevent camera tracking system component 6 from tipping over when cameras 46 is disposed over a patient, as illustrated in Figures 3A and 5.

Camera telescoping support 40 may support cameras 46 on the auxiliary tracking bar. In some embodiments, telescoping support 40 moves cameras 46 higher or lower in the vertical direction. Camera handle 48 may be attached to camera telescoping support 40 at any suitable location and configured to allow an operator to move camera tracking system component 6 into a planned position before a medical operation. In some embodiments, camera handle 48 is used to lower and raise camera telescoping support 40. Camera handle 48 may perform the raising and lowering of camera telescoping support 40 through the depression of a button, switch, lever, and/or any combination thereof.

Lower camera support arm 42 may attach to camera telescoping support 40 at any suitable location, in embodiments, as illustrated in Figure 1, lower camera support arm 42 may rotate three hundred and sixty degrees around telescoping support 40. This free rotation may allow an operator to position cameras 46 in any suitable location. Lower camera support arm 42 may connect to telescoping support 40 by any suitable mechanism. Lower camera support arm 42 may be used to provide support for cameras 46. Cameras 46 may be attached to lower camera support arm 42 by any suitable mechanism. Cameras 46 may pivot in any direction at the attachment area between cameras 46 and lower camera support arm 42. In embodiments a curved rail 44 may be disposed on lower camera support arm 42.

Curved rail 44 may be disposed at any suitable location on lower camera support arm 42. As illustrated in Figure 3A, curved rail 44 may attach to lower camera support arm 42 by any suitable mechanism. Curved rail 44 may be of any suitable shape, a suitable shape may be a crescent, circular, oval, elliptical, and/or any combination thereof. Cameras 46 may be moveably disposed along curved rail 44. Cameras 46 may attach to curved rail 44 by, for example, rollers, brackets, braces, motors, and/or any combination thereof. Motors and rollers, not illustrated, may be used to move cameras 46 along curved rail 44. As illustrated in Figure 3A, during a medical procedure, if an object prevents cameras 46 from viewing one or more DRAs being tracked, the motors may responsively move cameras 46 along curved rail 44. This motorized movement may allow cameras 46 to move to a new position that is no longer obstructed by the object without moving camera tracking system component 6. While cameras 46 is obstructed from viewing one or more tracked DRAs, camera tracking system component 6 may send a stop signal to a surgical robot 4, XR headset, display 34, and/or a tablet. The stop signal may prevent SCARA 24 from moving until cameras 46 has reacquired tracked DRAs 52 and/or can warn an operator wearing the XR headset and/or viewing the display 34 and/or the tablet. This SCARA 24 can be configured to respond to receipt of a stop signal by stopping further movement of the base and/or end effector coupler 22 until the camera tracking system can resume tracking of DRAs.

The end effector coupler 22 can include a load cell interposed between a saddle join and a connected passive end effector. The load cell may be any suitable instrument used to detect and measure forces. In some examples, the load cell may be a six axis load cell, a three-axis load cell or a uniaxial load cell. The load cell may be used to track the force applied to end effector coupler 22. In some embodiments the load cell may communicate with a plurality of motors 850, 851, 852, 853, and/or 854. As the load cell senses force, information as to the amount of force applied may be distributed to a controller 846 (Fig. 8). Controller 846 may take the force information from the load cell and process it with a switch algorithm. The switch algorithm is used by the controller 846 to control a motor driver 842. The motor driver 842 controls operation of one or more of the motors. Motor driver 842 may direct a specific motor to produce, for example, an equal amount of force measured by load cell through the motor. In some embodiments, the force produced may come from a plurality of motors, e.g., 850-854, as directed by controller 846. Additionally, motor driver 842 may receive input from controller 846. Controller 846 may receive information from load cell as to the direction of force sensed by load cell. Controller 846 may process this information using a motion controller algorithm. The algorithm may be used to provide information to specific motor drivers 842. To replicate the direction of force, controller 846 may activate and/or deactivate certain motor drivers 842. Controller 846 may control one or more motors, e.g. one or more of 850-854, to induce motion of passive end effector 1000 in the direction of force sensed by load cell. This force-controlled motion may allow an operator to move SCARA 24 and passive end effector 1000 effortlessly and/or with very little resistance. Movement of passive end effector 1000 can be performed to position passive end effector 1000 in any suitable pose (i.e., location and angular orientation relative to defined three-dimensional (3D) orthogonal reference axes) for use by medical personnel.

Figures 3B and 3C illustrate a front view and isometric view of another camera tracking system component 6' which may be used with the surgical system of Figure 1 or may be used independent of a surgical robot. For example, the camera tracking system component 6' may be used for providing navigated surgery without use of robotic guidance. One of the differences between the camera tracking system component 6' of Figures 3B and 3C and the camera tracking system component 6 of Figure 3A, is that the camera tracking system component 6' of Figures 3B and 3C includes a housing that transports the computer platform 910. The computer platform 910 can be configured to perform camera tracking operations to track DRAs, perform navigated surgery operations that provide surgical navigation information to a display device, e.g., XR headset and/or other display device, and perform other computational operations disclosed herein. The computer platform 910 can therefore include a navigation computer, such as one or more of the navigation computers of Figures 14.

Figure 6 illustrates a block diagram view of the components of the surgical system of Figure 5 used for the medical operation. Referring to Figure 6, the tracking cameras 46 on the auxiliary tracking bar has a navigation field-of-view 600 in which the pose (e.g., position and orientation) of the reference array 602 attached to the patient, the reference array 604 attached to the surgical instrument, and the robot arm 20 are tracked. The tracking cameras 46 may be part of the camera tracking system component 6' of Figures 3B and 3C, which includes the computer platform 910 configured to perform the operations described below. The reference arrays enable tracking by reflecting light in known patterns, which are decoded to determine their respective poses by the tracking subsystem of the surgical robot 4. If the line-of-sight between the patient reference array 602 and the tracking cameras 46 in the auxiliary tracking bar is blocked (for example, by a medical personnel, instrument, etc.), further navigation of the surgical instrument may not be able to be performed and a responsive notification may temporarily halt further movement of the robot arm 20 and surgical robot 4, display a warning on the display 34, and/or provide an audible warning to medical personnel. The display 34 is accessible to the surgeon 610 and assistant 612 but viewing requires a head to be turned away from the patient and for eye focus to be changed to a different distance and location. The navigation software may be controlled by a tech personnel 614 based on vocal instructions from the surgeon.

Figure 7 illustrates various display screens that may be displayed on the display 34 of Figures 5 and 6 by the surgical robot 4 when using a navigation function of the surgical system 2. The display screens can include, without limitation, patient radiographs with overlaid graphical representations of models of instruments that are positioned in the display screens relative to the anatomical structure based on a developed surgical plan and/or based on poses of tracked reference arrays, various user selectable menus for controlling different stages of the surgical procedure and dimension parameters of a virtually projected implant (e.g. length, width, and/or diameter).

For navigated surgery, various processing components (e.g., computer platform 910) and associated software described below are provided that enable pre-operatively planning of a surgical procedure, e.g., implant placement, and electronic transfer of the plan to computer platform 910 to provide navigation information to one or more users during the planned surgical procedure.

For robotic navigation, various processing components (e.g., computer platform 910) and associated software described below are provided that enable pre-operatively planning of a surgical procedure, e.g., implant placement, and electronic transfer of the plan to the surgical robot 4. The surgical robot 4 uses the plan to guide the robot arm 20 and connected end effector 26 to provide a target pose for a surgical tool relative to a patient anatomical structure for a step of the planned surgical procedure.

Various embodiments below are directed to using one or more XR headsets that can be worn by the surgeon 610, the assistant 612, and/or other medical personnel to provide an improved user interface for receiving information from and/or providing control commands to the surgical robot, the camera tracking system component 6/6', and/or other medical equipment in the operating room.

Figure 8 illustrates a block diagram of some electrical components of the surgical robot 4 according to some embodiments of the present disclosure. Referring to Figure 8, a load cell (not shown) may be configured to track force applied to end effector coupler 22. In some embodiments the load cell may communicate with a plurality of motors 850, 851, 852, 853, and/or 854. As load cell senses force, information as to the amount of force applied may be distributed from a switch array and/or a plurality of switch arrays to a controller 846. Controller 846 may take the force information from load cell and process it with a switch algorithm. The switch algorithm is used by the controller 846 to control a motor driver 842. The motor driver 842 controls operation of one or more of the motors 850, 851, 852, 853, and 854. Motor driver 842 may direct a specific motor to produce, for example, an equal amount of force measured by load cell through the motor. In some embodiments, the force produced may come from a plurality of motors, e.g., 850-854, as directed by controller 846. Additionally, motor driver 842 may receive input from controller 846. Controller 846 may receive information from load cell as to the direction of force sensed by load cell. Controller 846 may process this information using a motion controller algorithm. The algorithm may be used to provide information to specific motor drivers 842. To replicate the direction of force, controller 846 may activate and/or deactivate certain motor drivers 842. Controller 846 may control one or more motors, e.g. one or more of 850-854, to induce motion of end effector 26 in the direction of force sensed by load cell. This force-controlled motion may allow an operator to move SCARA 24 and end effector 26 effortlessly and/or with very little resistance. Movement of end effector 26 can be performed to position end effector 26 in any suitable pose (i.e., location and angular orientation relative to defined three-dimensional (3D) orthogonal reference axes) for use by medical personnel.

Activation assembly 60, best illustrated in Figure 5, may form of a bracelet that wraps around end effector coupler 22. The activation assembly 60 may be located on any part of SCARA 24, any part of end effector coupler 22, may be worn by medical personnel (and communicate wirelessly), and/or any combination thereof. Activation assembly 60 may comprise of a primary button and a secondary button.

Depressing primary button may allow an operator to move SCARA 24 and end effector coupler 22. According to one embodiment, once set in place, SCARA 24 and end effector coupler 22 may not move until an operator programs surgical robot 4 to move SCARA 24 and end effector coupler 22, or is moved using primary button. In some examples, it may require the depression of at least two non-adjacent primary activation switches before SCARA 24 and end effector coupler 22 will respond to operator commands. Depression of at least two primary activation switches may prevent the accidental movement of SCARA 24 and end effector coupler 22 during a medical procedure.

Activated by primary button, load cell may measure the force magnitude and/or direction exerted upon end effector coupler 22 by an operator, i.e. medical personnel. This information may be transferred to one or more motors, e.g. one or more of 850-854, within SCARA 24 that may be used to move SCARA 24 and end effector coupler 22. Information as to the magnitude and direction of force measured by load cell may cause the one or more motors, e.g. one or more of 850-854, to move SCARA 24 and end effector coupler 22 in the same direction as sensed by the load cell. This force-controlled movement may allow the operator to move SCARA 24 and end effector coupler 22 easily and without large amounts of exertion due to the motors moving SCARA 24 and end effector coupler 22 at the same time the operator is moving SCARA 24 and end effector coupler 22.

In some examples, a secondary button may be used by an operator as a "selection" device. During a medical operation, surgical robot 4 may notify medical personnel to certain conditions by the XR headset(s) 920, display 34 and/or light indicator 28. The XR headset(s) 920 are each configured to display images on a see-through display screen to form an extended reality image that is overlaid on real-world objects viewable through the see-through display screen. Medical personnel may be prompted by surgical robot 4 to select a function, mode, and/or asses the condition of surgical system 2. Depressing secondary button a single time may activate certain functions, modes, and/or acknowledge information communicated to medical personnel through the XR headset(s) 920, display 34 and/or light indicator 28. Additionally, depressing the secondary button multiple times in rapid succession may activate additional functions, modes, and/or select information communicated to medical personnel through the XR headset(s) 920, display 34 and/or light indicator 28.

With further reference to Figure 8, electrical components of the surgical robot 4 include platform subsystem 802, computer subsystem 820, motion control subsystem 840, and tracking subsystem 830. Platform subsystem 802 includes battery 806, power distribution module 804, connector panel 808, and charging station 810. Computer subsystem 820 includes computer 822, display 824, and speaker 826. Motion control subsystem 840 includes driver circuit 842, motors 850, 851, 852, 853, 854, stabilizers 855, 856, 857, 858, end effector connector 844, and controller 846. Tracking subsystem 830 includes position sensor 832 and camera converter 834. Surgical robot 4 may also include a removable foot pedal 880 and removable tablet computer 890.

Input power is supplied to surgical robot 4 via a power source which may be provided to power distribution module 804. Power distribution module 804 receives input power and is configured to generate different power supply voltages that are provided to other modules, components, and subsystems of surgical robot 4. Power distribution module 804 may be configured to provide different voltage supplies to connector panel 808, which may be provided to other components such as computer 822, display 824, speaker 826, driver 842 to, for example, power motors 850-854 and end effector coupler 844, and provided to camera converter 834 and other components for surgical robot 4. Power distribution module 804 may also be connected to battery 806, which serves as temporary power source in the event that power distribution module 804 does not receive power from an input power. At other times, power distribution module 804 may serve to charge battery 806.

Connector panel 808 may serve to connect different devices and components to surgical robot 4 and/or associated components and modules. Connector panel 808 may contain one or more ports that receive lines or connections from different components. For example, connector panel 808 may have a ground terminal port that may ground surgical robot 4 to other equipment, a port to connect foot pedal 880, a port to connect to tracking subsystem 830, which may include position sensor 832, camera converter 834, and DRA tracking cameras 870. Connector panel 808 may also include other ports to allow USB, Ethernet, HDMI communications to other components, such as computer 822. In accordance with some embodiments, the connector panel 808 can include a wired and/or wireless interface for operatively connecting one or more XR headsets 920 to the tracking subsystem 830 and/or the computer subsystem 820.

Control panel 816 may provide various buttons or indicators that control operation of surgical robot 4 and/or provide information from surgical robot 4 for observation by an operator. For example, control panel 816 may include buttons to power on or off surgical robot 4, lift or lower vertical column 16, and lift or lower stabilizers 855-858 that may be designed to engage casters 12 to lock surgical robot 4 from physically moving. Other buttons may stop surgical robot 4 in the event of an emergency, which may remove all motor power and apply mechanical brakes to stop all motion from occurring. Control panel 816 may also have indicators notifying the operator of certain system conditions such as a line power indicator or status of charge for battery 806. In accordance with some embodiments, one or more XR headsets 920 may communicate, e.g. via the connector panel 808, to control operation of the surgical robot 4 and/or to received and display information generated by surgical robot 4 for observation by persons wearing the XR headsets 920.

Computer 822 of computer subsystem 820 includes an operating system and software to operate assigned functions of surgical robot 4. Computer 822 may receive and process information from other components (for example, tracking subsystem 830, platform subsystem 802, and/or motion control subsystem 840) in order to display information to the operator. Further, computer subsystem 820 may provide output through the speaker 826 for the operator. The speaker may be part of the surgical robot, part of an XR headset 920, or within another component of the surgical system 2. The display 824 may correspond to the display 34 shown in Figures 1 and 2.

Tracking subsystem 830 may include position sensor 832 and camera converter 834. Tracking subsystem 830 may correspond to the camera tracking system component 6 of Figure 3. The DRA tracking cameras 870 operate with the position sensor 832 to determine the pose of DRAs 52. This tracking may be conducted in a manner consistent with the present disclosure including the use of infrared or visible light technology that tracks the location of active or passive elements of DRAs 52, such as LEDs or reflective fiducials (also called markers), respectively.

The location, orientation, and position of structures having these types of markers, such as DRAs 52, is provided to computer 822 and which may be shown to an operator on display 824. For example, as shown in Figures 4 and 5, a surgical saw 1040 having a DRA 52 or which is connected to an end effector coupler 22 having a DRA 52 tracked in this manner (which may be referred to as a navigational space) may be shown to an operator in relation to a three dimensional image of a patient's anatomical structure.

Functional operations of the tracking subsystem 830 and the computer subsystem 820 can be included in the computer platform 910, which can be transported by the camera tracking system component 6' of Figures 3A and 3B. The tracking subsystem 830 can be configured to determine the poses, e.g., location and angular orientation of the tracked DRAs. The computer platform 910 can also include a navigation controller that is configured to use the determined poses to provide navigation information to users that guides their movement of tracked tools relative to position-registered patient images and/or tracked anatomical structures during a planned surgical procedure. The computer platform 910 can display information on the display of Figures 3B and 3C and/or to one or more XR headsets 920. The computer platform 910, when used with a surgical robot, can be configured to communicate with the computer subsystem 820 and other subsystems of Figure 8 to control movement of the end effector 26. For example, as will be explained below the computer platform 910 can generate a graphical representation of a patient's anatomical structure, surgical tool, user's hand, etc. with a displayed size, shape, color, and/or pose that is controlled based on the determined pose(s) of one or more the tracked DRAs, and which the graphical representation that is displayed can be dynamically modified to track changes in the determined poses over time.

Motion control subsystem 840 may be configured to physically move vertical column 16, upper arm 18, lower arm 20, or rotate end effector coupler 22. The physical movement may be conducted through the use of one or more motors 850-854. For example, motor 850 may be configured to vertically lift or lower vertical column 16. Motor 851 may be configured to laterally move upper arm 18 around a point of engagement with vertical column 16 as shown in Figure 2. Motor 852 may be configured to laterally move lower arm 20 around a point of engagement with upper arm 18 as shown in Figure 2. Motors 853 and 854 may be configured to move end effector coupler 22 to provide translational movement and rotation along in about three-dimensional axes. The computer platform 910 shown in Figure 9 can provide control input to the controller 846 that guides movement of the end effector coupler 22 to position a passive end effector, which is connected thereto, with a planned pose (i.e., location and angular orientation relative to defined 3D orthogonal reference axes) relative to an anatomical structure that is to be operated on during a planned surgical procedure. Motion control subsystem 840 may be configured to measure position of the end effector coupler 22 and/or the end effector 26 using integrated position sensors (e.g. encoders).

Figure 9 illustrates a block diagram of components of a surgical system that includes imaging devices (e.g., C-Arm, O-Arm, etc.) connected to a computer platform 910 which can be operationally connected to a camera tracking system component 6 (Fig. 3A) or 6' (Figs. 3B, 3C) and/or to surgical robot 4 according to some embodiments of the present disclosure. Alternatively, at least some operations disclosed herein as being performed by the computer platform 910 may additionally or alternatively be performed by components of a surgical system.

Referring to Figure 9, the computer platform 910 includes a display 912, at least one processor circuit 914 (also referred to as a processor for brevity), at least one memory circuit 916 (also referred to as a memory for brevity) containing computer readable program code 918, and at least one network interface 902 (also referred to as a network interface for brevity). The display 912 may be part of an XR headset 920 in accordance with some embodiments of the present disclosure. The network interface 902 can be configured to connect to a C-Arm imaging device 104, an O-Arm imaging device 106, another medical imaging device, an image database 950 containing patient medical images, components of the surgical robot 4, and/or other electronic equipment.

When used with a surgical robot 4, the display 912 may correspond to the display 34 of Figure 2 and/or the tablet 890 of Figure 8 and/or the XR headset 920 that is operatively connected to the surgical robot 4, the network interface 902 may correspond to the platform network interface 812 of Figure 8, and the processor 914 may correspond to the computer 822 of Figure 8. The network interface 902 of the XR headset 920 may be configured to communicate through a wired network, e.g., thin wire ethernet, and/or through wireless RF transceiver link according to one or more wireless communication protocols, e.g., WLAN, 3GPP 4G and/or 5G (New Radio) cellular communication standards, etc.

The processor 914 may include one or more data processing circuits, such as a general purpose and/or special purpose processor, e.g., microprocessor and/or digital signal processor. The processor 914 is configured to execute the computer readable program code 918 in the memory 916 to perform operations, which may include some or all of the operations described herein as being performed for surgery planning, navigated surgery, and/or robotic surgery.

The processor 914 can operate to display on the display device 912 an image of a bone that is received from one of the imaging devices 104 and 106 and/or from the image database 950 through the network interface 902. The processor 914 receives an operator's definition of where an anatomical structure, i.e. one or more bones, shown in one or more images is to be cut, such as by an operator touch selecting locations on the display 912 for planned surgical cuts or using a mouse-based cursor to define locations for planned surgical cuts.

computer platform 910 can be configured to provide surgery planning functionality. The processor 914 can operate to display on the display device 912 and/or on the XR headset 920 an image of an anatomical structure, e.g., vertebra, that is received from one of the imaging devices 104 and 106 and/or from the image database 950 through the network interface 902. The processor 914 receives an operator's definition of where the anatomical structure shown in one or more images is to have a surgical procedure, e.g., screw placement, such as by the operator touch selecting locations on the display 912 for planned procedures or using a mouse-based cursor to define locations for planned procedures. When the image is displayed in the XR headset 920, the XR headset can be configured to sense in gesture-based commands formed by the wearer and/or sense voice based commands spoken by the wearer, which can be used to control selection among menu items and/or control how objects are displayed on the XR headset 920 as will be explained in further detail below.

The computer platform 910 can be configured to enable anatomy measurement, which can be particularly useful for knee surgery, like measurement of various angles determining center of hip, center of angles, natural landmarks (e.g. transepicondylar line, Whitesides line, posterior condylar line etc.), etc. Some measurements can be automatic while some others involve human input or assistance. This computer platform 910 can allow an operator to choose the correct implant for a patient, including choice of size and alignment. As will be explained further below, a ML guidance system 1220 (Fig. 12) provides guidance to a user during pre-operative planning and during intra-operative surgical execution of the surgical plan. The ML guidance system enables automatic or semi-automatic (involving human input) selection of implant(s) and generation of the surgical plan.

The surgical planning computer 910 enables automatic or semi-automatic segmentation (image processing) for CT images or other medical images. The surgical plan for a patient may be stored in a central database 1210 (Fig. 12) for retrieval by the surgical robot 800. During the surgery, the surgeon will choose which cut to make (e.g. posterior femur, proximal tibia etc.) using a computer screen (e.g. touchscreen) or augmented reality interaction via, e.g., a head-mounted display. The surgical robot 4 may automatically move the surgical saw 1040 to a planned position so that a target plane of planned cut is optimally placed within a workspace of the passive end effector interconnecting the surgical saw 1040 and the robot arm 20.

During TKA, for example, a surgeon may choose which cut to make (e.g. posterior femur, proximal tibia etc.) using a computer screen (e.g. touchscreen) or extended reality (XR) interaction (e.g., hand gesture based commands and/or voice based commands) via, e.g., the XR headset 920. The computer platform 910 can generate navigation information which provides visual guidance to the surgeon for performing the surgical procedure. When used with the surgical robot 4, the computer platform 910 can provide guidance that allows the surgical robot 4 to automatically or semi-automatically move the end effector 26 to a target pose so that the surgical tool is aligned with a target location to perform the surgical procedure on an anatomical structure.

In some embodiments, the computer platform 910 can use two DRAs to tracking patient anatomy position: one on patient tibia and one on patient femur. The platform 900 may use standard navigated instruments for the registration and checks (e.g. a pointer similar to the one used in Globus ExcelsiusGPS system for spine surgery). DRAs allowing for detection of DRAs movement in reference to tracked anatomy can be used as w

A particular difficulty in knee surgery is how to plan the position of the implant in the knee and many surgeons struggle with to do it on a computer screen which is a 2D representation of 3D anatomy. The system 900 could address this problem by using the XR headset 920 to display a three-dimensional (3D) computer generated representation of the overlaid on the real patient knee. The computer generated representation is scaled and posed relative to the patient on the display screen under guidance of the computer platform 910, and the pose can be manipulated by a surgeon while viewed through the XR headset 920. A surgeon may, for example, manipulate the displayed computer-generated representation of the anatomical structure, the implant, a surgical tool, etc., using hand gesture based commands and/or voice based commands that are sensed by the XR headset 920.

For example, during the pre-operative stage a surgeon can view a displayed virtual handle on a virtual implant, and can manipulate (e.g., grab and move) the virtual handle to move the virtual implant to a desired pose and adjust a planned implant placement relative to a graphical representation of the patient's knee or other anatomical structure. Afterward, during surgery, the computer platform 910 could display navigation information through the XR headset 920 that facilitates the surgeon's ability to more accurately follow the surgical plan to insert the implant and/or to perform another surgical procedure on the knee. When the surgical procedure involves bone removal, the progress of bone removal, e.g., depth of cut, can be displayed in real-time through the XR headset 920. Other features that may be displayed through the XR headset 920 can include, without limitation, gap or ligament balance along a range of joint motion, contact line on the implant along the range of joint motion, ligament tension and/or laxity through color or other graphical renderings, etc.

The computer platform 910, in some embodiments, can allow planning for use of standard surgical tools and/or implants, e.g., posterior stabilized implants and cruciate retaining implants, cemented and cementless implants, revision systems for surgeries related to, for example, total or partial knee and/or hip replacement and/or trauma.

The computer platform 910 may graphically illustrate one or more cutting planes intersecting the displayed anatomical structure at the locations selected by the operator for cutting the anatomical structure. The computer platform 910 also determines one or more sets of angular orientations and locations where the end effector coupler 22 must be positioned so a cutting plane of the surgical saw will be aligned with a target plane to perform the operator defined cuts, and stores the sets of angular orientations and locations as data in a surgical plan data structure. The computer platform 910 uses the known range of movement of the tool attachment mechanism of the passive end effector to determine where the end effector coupler 22 attached to the robot arm 20 needs to be positioned.

The computer subsystem 820 of the surgical robot 800 receives data from the surgical plan data structure and receives information from the camera tracking system component 6 indicating a present pose of an anatomical structure that is to be cut and indicating a present pose of the passive end effector and/or surgical saw tracked through DRAs. The computer subsystem 820 determines a pose of the target plane based on the surgical plan defining where the anatomical structure is to be cut and based on the pose of the anatomical structure, The computer subsystem 820 generates steering information based on comparison of the pose of the target plane and the pose of the surgical saw. The steering information indicates where the passive end effector needs to be moved so the cutting plane of the saw blade becomes aligned with the target plane and the saw blade becomes positioned a distance from the anatomical structure to be cut that is within the range of movement of the tool attachment mechanism of the passive end effector.

As explained above, a surgical robot includes a robot base, a robot arm connected to the robot base, and at least one motor operatively connected to move the robot arm relative to the robot base. The surgical robot also includes at least one controller, e.g. the computer subsystem 820 and the motion control subsystem 840, connected to the at least one motor and configured to perform operations.

As will be explained in further detail below with regard to Figures 10 and 11, a passive end effector includes a base configured to attach to an activation assembly of the robot arm, a first mechanism, and a second mechanism. The first mechanism extends between a rotatable connection to the base and a rotatable connection to a tool attachment mechanism. The second mechanism extends between a rotatable connection to the base and a rotatable connection to the tool attachment mechanism. The first and second mechanisms pivot about the rotatable connections which may be configured to constrain movement of the tool attachment mechanism to a range of movement within a working plane. The rotatable connections may be pivot joints allowing 1 degree-of-freedom (DOF) motion, universal joints allowing 2 DOF motions, or ball joints allowing 3 DOF motions. The tool attachment mechanism is configured to connect to the surgical saw comprising a saw blade for cutting. The first and second mechanisms may be configured to constrain a cutting plane of the saw blade to be parallel to the working plane.

In some embodiments, the operations performed by the at least one controller of the surgical robot also includes controlling movement of the at least one motor based on the steering information to reposition the passive end effector so the cutting plane of the saw blade becomes aligned with the target plane and the saw blade becomes positioned the distance from the anatomical structure to be cut that is within the range of movement of the tool attachment mechanism of the passive end effector. The steering information may be displayed to guide an operator's movement of the surgical saw and/or may be used by the at least one controller to automatically move the surgical saw.

In one embodiment, the operations performed by the at least one controller of the surgical robot also includes providing the steering information to a display device for display to guide operator movement of the passive end effector so the cutting plane of the saw blade becomes aligned with the target plane and so the saw blade becomes positioned the distance from the anatomical structure, which is to be cut, that is within the range of movement of the tool attachment mechanism of the passive end effector.

For example, the steering information may be displayed on the XR-headset 920 which projects images onto a see-through display screen which forms an XR image that is overlaid on real-world objects viewable through the see-through display screen. The operations may display a graphical representation of the target plane with a pose overlaid on a bone and with a relative orientation there between corresponding to the surgical plan for how the bone is planned to be cut. The operations may alternatively or additionally display a graphical representation of the cutting plane of the saw blade so that an operator may more easily align the cutting plane with the planned target plane for cutting the bone. The operator may thereby visually observe and perform movements to align the cutting plane of the saw blade with the target plane so the saw blade becomes positioned at the planned pose relative to the bone and within a range of movement of the tool attachment mechanism of the passive end effector.

An automated imaging system can be used in conjunction with the surgical planning computer 910 and/or the surgical system 2 to acquire pre-operative, intra-operative, post-operative, and/or real-time image data of a patient. In some embodiments, the automated imaging system is a C-arm imaging device or an O-arm^{®}. (O-arm^{®} is copyrighted by Medtronic Navigation, Inc. having a place of business in Louisville, Colo., USA) It may be desirable to take x-rays of a patient from a number of different positions, without the need for frequent manual repositioning of the patient which may be required in an x-ray system. C-arm x-ray diagnostic equipment may solve the problems of frequent manual repositioning and may be well known in the medical art of surgical and other interventional procedures. A C-arm includes an elongated C-shaped member terminating in opposing distal ends of the "C" shape. C-shaped member is attached to an x-ray source and an image receptor. The space within C-arm of the arm provides room for the physician to attend to the patient substantially free of interference from the x-ray support structure.

The C-arm is mounted to enable rotational movement of the arm in two degrees of freedom, (i.e. about two perpendicular axes in a spherical motion). C-arm is slidably mounted to an x-ray support structure, which allows orbiting rotational movement of the C-arm about its center of curvature, which may permit selective orientation of x-ray source and image receptor vertically and/or horizontally. The C-arm may also be laterally rotatable, (i.e. in a perpendicular direction relative to the orbiting direction to enable selectively adjustable positioning of x-ray source and image receptor relative to both the width and length of the patient). Spherically rotational aspects of the C-arm apparatus allow physicians to take x-rays of the patient at an optimal angle as determined with respect to the particular anatomical condition being imaged.

An O-arm^{®} includes a gantry housing which may enclose an image capturing portion, not illustrated. The image capturing portion includes an x-ray source and/or emission portion and an x-ray receiving and/or image receiving portion, which may be disposed about one hundred and eighty degrees from each other and mounted on a rotor relative to a track of the image capturing portion. The image capturing portion may be operable to rotate three hundred and sixty degrees during image acquisition. The image capturing portion may rotate around a central point and/or axis, allowing image data of the patient to be acquired from multiple directions or in multiple planes.

The O-arm^{®} with the gantry housing has a central opening for positioning around an object to be imaged, a source of radiation that is rotatable around the interior of gantry housing, which may be adapted to project radiation from a plurality of different projection angles. A detector system is adapted to detect the radiation at each projection angle to acquire object images from multiple projection planes in a quasi-simultaneous manner. The gantry may be attached to a support structure O-arm^{®} support structure, such as a wheeled mobile cart with wheels, in a cantilevered fashion. A positioning unit translates and/or tilts the gantry to a planned position and orientation, preferably under control of a computerized motion control system. The gantry may include a source and detector disposed opposite one another on the gantry. The source and detector may be secured to a motorized rotor, which may rotate the source and detector around the interior of the gantry in coordination with one another. The source may be pulsed at multiple positions and orientations over a partial and/or full three hundred and sixty degree rotation for multi-planar imaging of a targeted object located inside the gantry. The gantry may further comprise a rail and bearing system for guiding the rotor as it rotates, which may carry the source and detector. Both and/or either O-arm^{®} and C-arm may be used as automated imaging system to scan a patient and send information to the surgical system 2.

Images captured by the automated imaging system can be displayed a display device of the surgical planning computer 910, the surgical robot 800, and/or another component of the surgical system 2.

Various embodiments of passive end effectors that are configured for use with a surgical system are now described in the context of Figures 10 and 11.

As will be explained in further detail below, the various passive end effectors illustrated in Figures 10 and 11 each include a base, a first planer mechanism, and a second planner mechanism. The base is configured to attach to an end effector coupler (e.g., end effector coupler 22 in Figs. 4 and 5) of a robot arm (e.g., robot arm 18 in Figs 1 and 2) positioned by a surgical robot. Various clamping mechanisms may be used to firmly attach the base to the end effector coupler, removing backlash and ensuring suitable stiffness. The first mechanism extends between a rotatable connection to the base of a two and a rotatable connection to a tool attachment mechanism. The second mechanism extends between a rotatable connection to the base and a rotatable connection to the tool attachment mechanism. The first and second mechanisms pivot about the rotatable connections. The rotatable connections may be pivot joints allowing 1 degree-of-freedom (DOF) motion, universal joints allowing 2 DOF motions, or ball joints allowing 3 DOF motions. When pivot joints are used the first and second mechanisms can be configured to constrain movement of the tool attachment mechanism to a range of movement within a working plane. The tool attachment mechanism is configured to connect to a surgical saw having a saw blade that is configured to oscillate for cutting. The first and second mechanisms may be configured, e.g., via pivot joints having 1 DOF motion, to constrain a cutting plane of the saw blade to be parallel to the working plane. The tool attachment mechanism may connect to the surgical saw through various mechanisms that can include, but are not limited to, a screw, nut and bolt, clamp, latch, tie, press fit, or magnet. A DRA can be connected to the tool attachment mechanism or the surgical saw to enable tracking of a pose of the saw blade by the camera tracking system 6 (Fig. 3).

As explained above, a surgical system (e.g., surgical system 2 in Figs. 1 and 2) includes a surgical robot (e.g., surgical robot 4 in Figs. 1 and 2) and a tracking system (e.g., camera tracking system 6 in Figs. 1 and 3) that is configured to determine a pose of an anatomical structure that is to be cut by the saw blade and to determine a pose of the saw blade. The surgical robot includes a robot base, a robot arm that is rotatably connected to the robot base and configured to position the passive end effector. At least one motor is operatively connected to move the robot arm relative to the robot base. At least one controller is connected to the at least one motor and configured to perform operations that include determining a pose of a target plane based on a surgical plan defining where the anatomical structure is to be cut and based on the pose of the anatomical structure, where the surgical plan may be generated by the surgical planning computer 910 of Figure 9 based on input from an operator, e.g., surgeon or other surgery personnel. The operations further include generating steering information based on comparison of the pose of the target plane and the pose of the surgical saw. The steering information indicates where the passive end effector needs to be moved to position the working plane of the passive end effector so the cutting plane of the saw blade is aligned with the target plane.

In some further embodiments, the operations performed by the at least one controller further include controlling movement of the at least one motor based on the steering information to reposition the passive end effector so the cutting plane of the saw blade becomes aligned with the target plane and the saw blade becomes positioned a distance from the anatomical structure to be cut that is within the range of movement of the tool attachment mechanism of the passive end effector.

The operations may include providing the steering information to a display device for display to guide operator movement of the passive end effector so the cutting plane of the saw blade becomes aligned with the target plane and so the saw blade becomes positioned a distance from the anatomical structure, which is to be cut, that is within the range of movement of the tool attachment mechanism of the passive end effector.

As explained above, some surgical systems can include head-mounted display devices that can be worn by a surgeon, nurse practitioner, and/or other persons assisting with the surgical procedure. The surgical systems can display information that allows the wearer to position the passive end effector more accurately and/or to confirm that it has been positioned accurately with the saw blade aligned with the target plane for cutting a planned location on an anatomical structure. The operation to provide the steering information to the display device, may include configuring the steering information for display on a head-mounted display device having a see-through display screen that displays the steering information as an overlay on the anatomical structure that is to be cut to guide operator movement of the passive end effector so the cutting plane of the saw blade becomes aligned with the target plane and the saw blade becomes positioned the distance from the anatomical structure within the range of movement of the tool attachment mechanism of the passive end effector.

The operation to configure the steering information for display on the head-mounted display device, may include generating a graphical representation of the target plane that is displayed as an overlay anchored to and aligned with the anatomical structure that is to be cut, and generating another graphical representation of the cutting plane of the saw blade that is displayed as an overlay anchored to and aligned with the saw blade. A wearer may thereby move the surgical saw to provide visually observed alignment between the graphically rendered target plane and the graphically rendered cutting plane.

The operation to configure the steering information for display on the head-mounted display device, may include generating a graphical representation a depth of cut made by the saw blade into a graphical representation of the anatomical structure being cut. Thus, the wearer can use the graphical representation of depth of cut to better monitor how the saw blade is cutting through bone despite direct observation of the cutting being obstructed by tissue or other structure.

The tracking system can be configured to determine the pose of the anatomical structure that is to be cut by the saw blade based on determining a pose of tracking markers, e.g., DRAs, that are attached to the anatomical structure, and can be configured to determine a pose of the surgical saw based on determining a pose of tracking markers connected to at least one of the surgical saw and the passive end effector. The tracking system can be configured to determine the pose of the surgical saw based on rotary position sensors which are configured to measure rotational positions of the first and second mechanisms during movement of the tool attachment mechanism within the working plane. As explained above, position sensors may be directly connected to at least one joint of the passive end effector structure, but may also be positioned in another location in the structure and remotely measure the joint position by interconnection of a timing belt, a wire, or any other synchronous transmission interconnection. Additionally the pose of the saw blade can be determined based on the tracking markers attached to the structure base, position sensors in the passive structure and kinematic model of the structure.

The various passive end effectors disclosed herein can be sterilizable or non-sterile (covered by a sterile drape) passive 3DOF (Degree Of Freedom) mechanical structures allowing mechanical guidance of a surgical saw, such as a sagittal saw, along two translations in a plane parallel to the saw blade (defining the cut plane), and one rotation perpendicular to this cut plane (instrument orientation). During the surgery, the surgical robot 4 moves the end effector coupler 22, and the passive end effector and surgical saw attached there, automatically to a position close to a knee or other anatomical structure, so that all bone to be cut is within the workspace of the passive end effector. This position depends on the cut to be made and the surgery planning and implant construction. The passive end effector can have 3 DOF to guide sagittal saw on the cutting plane providing two translation (X and Y directions) and a rotation (around Z axis) as shown in Figure 10.

When the surgical robot 4 achieves a planned position, it holds the position (either on brakes or active motor control) and does not move during the particular bone cut. It is the passive end effector that allows movement of the saw blade of the surgical saw along the planned target plane. Such planar cuts are particularly useful for classical total knee arthroplasty where all bone cuts are planar. In partial knee arthroplasty there are special types of implants, called "on-lay" which can be in conjunction with saw-prepared bone surfaces. The various passive end effectors have mechanical structure that can ensure precision of guidance during cuts, with higher precision than classical jigs, and provide sufficient range of workspace range to cut all the bone that is planned and while provide sufficient transverse stiffness (corresponding to locked DOF) despite possibly significant amount of vibrations originating from the surgical saw in addition to forces applied by the surgeon and bone reactionary forces.

As the same time, it is preferable to measure the passive end effector position because it enables the surgical robot 4 to inform the surgeon how much bone has been removed (procedure advancement). One way to provide real-time information on bone removal is for the surgical robot 4 to measure where the saw blade passed in reference to the bone because the blade can pass only where the bone has been cut. To measure sawblade position a DRA can be mounted to the surgical saw and/or the passive end effector. This enables direct or indirect measurement of the saw position in 3D space. An alternative way to measure saw blade position is to integrate position (rotation or translation) sensors (e.g. encoders, resolvers) into position information of the passive end effector in order to calculate position of the saw blade using a mathematical model of a defined relationship between location of the passive end effector geometry and the saw blade.

In one embodiment, a conventional sagittal saw mechanism can be used with the computer platform 910 with little or no changes. The potential changes would involve adapting an external shield to enable easy attachment of the surgical saw to the passive end effector but would not necessarily involve changes in the internal mechanics. The passive end effector may be configured to connect to a conventional sagittal saw provided by, for example, DeSoutter company.

A first embodiment of a passive end effector is shown in Figure 10. Referring Figure 10, the passive end effector 1000 includes a base 1002 configured to attach to an end effector coupler (e.g., end effector coupler 22 in Figs. 4 and 5) of a robot arm (e.g., robot arm 18 in Figs 1 and 2) positioned by a surgical robot. The passive end effector 1000 further includes first and second mechanisms that extend between rotatable connections to the base 1002 and rotatable connections to a tool attachment mechanism. The rotatable connections may be pivot joints allowing 1 degree-of-freedom (DOF) motion, universal joints allowing 2 DOF motions, or ball joints allowing 3 DOF motions. The first and second mechanisms form a parallel architecture that positions the surgical saw rotation axis in the cut plane.

First and second link segments 1010a and 1020a form the first planer mechanism, and third and fourth link segments 1010b and 1020b form the second planner mechanism. The first link segment 1010a extends between a rotatable connection to a first location on the base 1002 and a rotatable connection to an end of the second link segment 1020a. The third link segment 1010b extends between a rotatable connection to a second location on the base 1002 and a rotatable connection to an end of the fourth link segment 1020b. The first and second locations on the base 1002 are spaced apart on opposite sides of a rotational axis of the base color to when rotated by the robot arm. The tool attachment mechanism is formed by a fifth link segment that extends between rotatable connections to distal ends of the second link segment 1020a and the fourth link segment 1020b relative to the base 1002. The first and second mechanisms (first and second link segments 1010a-1020a and third and fourth link segments 1010b-1020b) pivot about their rotatable connections to constrain movement of the tool attachment mechanism 1030 to a range of movement within a working plane. The tool attachment mechanism 1030 is configured to connect to a surgical saw 1040 having a saw blade 1042 that is configured to oscillate for cutting. The first and second mechanisms (first and second link segments 1010a-1020a and third and fourth link segments 1010b-1020b) may be configured, e.g., via pivot joints having 1 DOF motion, to constrain a cutting plane of the saw blade 1042 to be parallel to the working plane. The tool attachment mechanism 1030 may connect to the surgical saw 1040 through various mechanisms that can include, but are not limited to, a screw, nut and bolt, clamp, latch, tie, press fit, or magnet. A DRA 52 can be connected to the tool attachment mechanism 1030 or the surgical saw 1040 to enable tracking of a pose of the saw blade 1042 by the camera tracking system 6 (Fig. 3).

The passive end effector 1000 provides passive guidance of the surgical saw 1040 to constrain the saw blade 1042 to a defined cutting plane and reduce its mobility to three degrees of freedom (DOF): two translations Tx and Ty in a plane parallel to the cutting plane of the saw blade 1042; and one rotational Rz around an axis perpendicular to the cutting plane.

In some embodiments, a tracking system is configured to determine the pose of the saw blade 1042 based on rotary position sensors connected to the rotational joints of at least some of the link segments of the passive end effector 1000. The rotary position sensors are configured to measure rotational positions of the joined link segments during movement of the tool attachment mechanism within the working plane. For example, a rotary position sensor can be configured to measure rotation of the first link segment 1010a relative to the base 1002, another rotary position sensor can be configured to measure rotation of the second link segment 1020a relative to the first link segment 1010a, and another rotary position sensor can be configured to measure rotation of the tool attachment mechanism 1030 relative to the second link segment 1020a. The surgical saw 1040 can connected to have a fixed orientation relative to the tool attachment mechanism 1030. A serial kinematic chain of the passive end effector 1000 connecting the saw blade 1042 and the robot arm 22, having serialized link segments and pivoting joints, provides the required mobility to the surgical saw 1040. The position of the tip of the saw blade 1042 in the plane defined by the passive kinematic chain can be fully determined by the joint angles, sensed through the rotary position sensors, and the structural geometry of the interconnected link segments. Therefore, by measuring the relative angle between each connected link segment, for example along one or more interconnected paths between the base 1002 and the surgical saw 1040, the position of the tip of the saw blade 1042 in the cut space can be computed using the proposed forward kinematic model. When the position and orientation of robot arm 22 distal end position and orientation with respect to the bone is known, the position and orientation of the saw blade 1042 with respect to the bone can be computed and displayed as feedback to the surgeon.

Example types of rotary position sensors that can be used with passive end effectors herein can include, but are not limited to: potentiometers; optical encoder; capacitive encoder; rotary variable differential transformer (RVDT) sensor; linear variable differential transformer (LVDT) sensor; Hall effect sensor; and incoder sensor.

Another embodiment of a passive end effector is shown in Figure 11. The passive end effector 1100 includes a base 1102 that is configured to attach to an end effector coupler (e.g., end effector coupler 22 in Figs. 4 and 5) of a robot arm (e.g., robot arm 18 in Figs 1 and 2) positioned by a surgical robot. The passive end effector 1100 further includes a first link segment 1110 and a second link segment 1120. The first link segment 1110 extends between a rotatable connection to the base 1102 and a rotatable connection to one end of the second link segment 1120. Another end of the second link segment 1120 is rotatably connected to a tool attachment mechanism. One or more of the rotatable connections disclosed for this embodiment may be pivot joints allowing 1 DOF motion, universal joints allowing 2 DOF motions, or ball joints allowing 3 DOF motions.

### Example Surgical Procedure

An example surgical procedure using the surgical robot 4 in an Operating Room (OR) can include:
1. Surgery is pre-operatively planned based on medical images.
2. The surgical robot 4 system is outside the Operating Room (OR). The nurse brings the system to the OR when patient is being prepared for the surgery.
3. The nurse powers on the robot and deploys the robot arm. Nurse verifies precision of robotic and tracking systems.
4. In the case of a sterilized passive end effector, the scrub nurse puts a sterile drape on the robot arm and mounts the passive end effector with the sagittal saw on the robot arm. The scrub nurse locks the passive end effector with a lock mechanism. Scrub nurse attached DRAs to passive structure through the drape (if necessary). For a non-sterilized passive end effector, the drape is placed after attachment of the passive end effector on the robot arm, the DRAs are attached to the passive end effector with the drape intervening therebetween, and a sterile saw is attached to the passive end effector with the drape intervening therebetween.
5. The surgeon attaches reference arrays (e.g., DRAs or navigation markers) to the patient's bone(s), e.g., tibia and femur. The DRAs are registered with the camera tracking system 6 using, e.g., Horn algorithm, surface matching or other algorithms. A soft-tissue balance assessment may be performed, whereby the system allows surgeon to assess balance of soft tissue in the operating room, e.g., by tracking relative movement of femur and tibia when surgeon applies forces in different directions (e.g. varus/valgus stress). Soft-tissue balance information can be used to alter surgical plan (e.g. move implant parts, change implant type etc.).
6. When surgeon is ready to cut the bone, the scrub nurse brings the surgical robot 4 to the operating table close to the knee to be operated and stabilizes the surgical robot 4 on the floor. The system may operate to guide nurse in finding robot 4 position so that all cut planes are in robot and passive structure workspace.
7. The surgeon selects on the screen of the surgical robot 4 the different parameters according to the planning of the surgery to do the first cut (bone to be cut, cutting plan desired, etc.).
8. The surgical robot 4 automatically moves the robot arm 22 to reposition the passive end effector so the cutting plane of the saw blade becomes aligned with the target plane and the saw blade becomes positioned a distance from the anatomical structure to be cut that is within the range of movement of the tool attachment mechanism of the passive end effector.
9. The surgeon unlocks the passive end effector.
10. The surgeon performs the cut constrained to the cutting plane provide by the passive end effector. The surgical robot 4 may provide real-time display of the tracked location of the saw blade relative to bone so the surgeon can monitor progress of bone removal. The surgeon can then lock the passive end effector using the lock mechanism upon completion of the cut.
11. The surgeon selects on the screen the next cut to be executed and proceeds as before.
12. The surgeon may perform a trial implant placement and intermediate soft-tissue balance assessment and based thereon may change the implant plan and associated cuts.
13. Following completion of all cuts, the nurse removes the surgical robot 4 from the operating table and unmounts the passive end effector from the robot arm.
14. The surgeon places the implants and finishes the surgery.

It is noted that the passive end effector may require calibration through the surgical robot 4 and camera track system 6 to precisely define a plane to which the surgical saw is constrained with respect to the robot arm 22. This calibration may be performed by moving the surgical saw in space and measuring the corresponding position with the tracking camera to define the plane. Alternative, calibration can be performed through use of specific divots provided in the passive end effector which are touched with a navigation probe.

### MACHINE LEARNING SYSTEM FOR NAVIGATED ORTHOPEDIC SURGERIES:

As explained above, in Total Knee Arthroplasty (TKA) surgeries patient satisfaction rates have remained principally unchanged over decades despite innovations in implant designs, computer-assisted surgery (CAS) such as by a navigation system and/or a robot system, custom cutting templates, and customized implants. This suggests that there are problems that have not been addressed with previous medical procedures and related innovations.

Possible causes of such problems may include:
- Inappropriate planning:
   ∘ TKA surgeries for all patients have been planned to have 0°±3° Hip-Knee-Ankle (HKA) angle after the surgery. However, this non-customized HKA is not appropriate for everybody.
   ∘ Joint line after the surgery is targeted through surgery to result in perpendicular to knee mechanical axis, although naturally this line is not perpendicular.
- Inappropriate execution:
   ∘ It has been demonstrated that about 30% of manual surgeries attempt but do not achieve the goal of 0°±3° HKA. In CAS the error rate is smaller but still not zero.
- Not representative post-operative data collection: Patient Reported Outcome Measures (PROMs) and functional tests are not strongly correlated to patient satisfaction.
- High variability of post-operative follow-up costs. Recently it has been demonstrated that professional rehabilitation does not improve surgery outcomes in comparison to patient self-rehabilitation done at home.

There are potentially many variables that influence the outcome of the surgery:
- Planning: how to make adapt surgeries to be more patient-specific? How to consider current patient deformity from a model? What shall be target deformity correction?
- What implant type is the best for a selected patient? It is noted that there can be more than several dozens types of available implant types that a surgeon may be able to select among for a patient.
- Implants can include four elements (tibial, femoral, polyethylene (PE), and patella), three of these elements have 6 DOF positions and PE of different heights.

These variables result in a large number of possible combinations that a surgeon may need to select among for use in an orthopedic surgery for a selected patient.

Some embodiments of the present disclosure are directed to a surgical guidance system that includes a machine learning processing circuit that processes data obtained and/or reported during pre-operative, intra-operative, and post-operative stages of surgery for patients. Over time, the machine learning processing circuit trains a machine learning model based on historical correlations and/or other trends determined between, for example, the variables (metrics or other data) that have been selected by surgeons during the pre-operative stage, the tracked movements during navigated surgery, and the resulting outcomes for patients. The training can include adapting rules of an artificial intelligence (Al) algorithm, rules of one or more sets of decision operations, and/or weights and/or firing thresholds of nodes of a neural network mode, to drive one or more defined key performance surgical outcomes toward one or more defined thresholds or other rule(s) being satisfied. The surgical guidance system processes pre-operative data for a new patent's characteristics through the machine learning model to provide navigated guidance to a surgeon during the pre-operative stage when generating a surgical plan with implant selection. The surgical plan can be provided to a navigation system to provide guidance to the surgeon during the intro-operative stage execution of the surgical plan and may be further provided to a surgical robot to control movements of a robot arm that assists the surgeon.

Figure 12 illustrates a navigated surgery workflow which uses a surgical guidance system 1220 configured in accordance with some embodiments. Referring to Figure 12, three stages of workflow are illustrated: pre-operative stage 1200; intra-operative stage 1202; and post-operative stage 1204: During the pre-operative stage 1200, a user (e.g., surgeon) generates a surgical plan (case) based on analyzed patient images with assistance from the surgical guidance system 1220. During the intra-operative stage 1202, the user is provided navigated assisted by the surgical guidance system 1220 which may include operation of a surgical robot 4 for precise plan execution. During the post-operative stage 1204, post-operative feedback data characterizing surgery outcomes is collected by the surgical guidance system 1220, such as by patient measurements and/or patient surveys, etc. Data obtained across all phases 1200 -1204 can be stored in a central database 1210 for use by the surgical guidance system 1220 to train a machine learning model of a machine learning processing circuit 1222. The machine learning model can include artificial intelligence (Al) processes, neural network components, etc. The machine learning model is trained over time and used to generate surgical plans that result in improved surgical outcomes.

The example surgical guidance system 1220 shown in Figure 12 includes a pre-operative planning component 1224, an intra-operative guidance component 1226, a machine learning processing circuit 1222, and a feedback training component 1228.

As will be explained in further detail below, the feedback training component 1228 is configured to obtain post-operative feedback data provided by distributed networked computers regarding surgical outcomes for a plurality of patients, and to train a machine learning model based on the post-operative feedback data. Although Figure 12 shows a single computer, e.g., smart phone, providing post-operative feedback data during the post-operative stage 1204 through one or more networks 1230 (e.g., public (Internet) networks and or private networks) to the surgical guidance system 1220 for storage in the central database 1210, it is to be understood that numerous network computers (e.g., hundreds of computers) would provide post-operative feedback data for each of many patients to the surgical guidance system 1220 (i.e., to the feedback training component 1228) for use in training the machine learning model. Moreover, as explained in further detail below, the feedback training component 1228 can further train the machine learning model based on pre-operative data obtained during the pre-operative stage 1200 for numerous patients and based on intra-operative data obtained during the intra-operative stage 1202 for numerous patients. For example, the training can include adapting rules of an Al algorithm, rules of one or more sets of decision operations, and/or weights and/or firing thresholds of nodes of a neural network mode, to drive one or more defined key performance surgical outcomes indicated by the pre-operative data and/or the intra-operative data toward one or more defined thresholds or other rule(s) being satisfied.

A pre-operative planning component 1224 obtains pre-operative data from one of the distributed network computers characterizing a defined patient, and generates a surgical plan for the defined patient based on processing the pre-operative data through the machine learning model. The pre-operative planning component 1224 provides the surgical plan to a display device for review by a user. Accordingly, the pre-operative planning component 1224 of the machine learning processing circuit 1222 generates a surgical plan for a defined patient using the machine learning model which has been trained based on the post-operative feedback data regarding surgical outcomes for the plurality of patients. The training of the machine learning model can be repeated as more post-operative feedback is obtained by the feedback training component 1228 so that the surgical plans that are generated will result in more continuous improvement of the resulting surgical outcomes for patients.

Figure 13 illustrates a block diagram of the surgical guidance system 1220 with associated data flows during the pre-operative, intra-operative, and post-operative stages, and shows surgical guidance being provided to user displays and to a robot surgery system.

Referring to Figure 13, the surgical guidance system 1220 includes the feedback training component 1228, the pre-operative planning component 1224, and the intra-operative guidance component 1226. The surgical guidance system 1220 also includes machine learning processing circuit 122 that includes machine learning module 1300, which may include an artificial intelligence and/or neural network component 1310 as explained in further detail below.

The feedback training component 1228 is configured to obtain post-operative stage feedback data provided by distributed networked computers regarding surgical outcomes for a plurality of patients. The feedback training component 1228 may also be configured to obtain pre-operative stage data and/or inter-operative stage data. The feedback training component 1228 uses the obtained data to train the machine learning model 1300.

In some embodiments, the feedback training component 1228 is configured to train the machine learning model 1300 based on the post-operative feedback data comprising at least one of: joint kinematics measurements; soft tissue balance measurements; deformity correction measurements; joint line measurements; and patient reported outcome measures.

In some additional or alternative embodiments, the feedback training component 1228 is configured to train the machine learning model 1300 based on at least one of: data indicating deviation between joint kinematics measurements of the defined patient during pre-operative stage compared to during post-operative stage; data indicating deviation between tissue balance measurements of the defined patient during pre-operative stage compared to during post-operative stage; data indicating deviation between deformity correction planned for the defined patient during pre-operative stage compared to deformity correction measured for the defined patient during post-operative stage; and data indicating deviation between joint line measurements of the defined patient during pre-operative stage compared to during post-operative stage.

In some additional or alternative embodiments, the feedback training component 1228 is configured to train the machine learning model 1300 based on the post-operative feedback data comprising at least one of: data indicating deviation of a surgical saw cutting plane measured during surgery from a surgical saw cutting plane defined by a surgical plan; data indicating deviation of surgical saw motion measurements during surgery from surgical saw motion defined by a surgical plan; data indicating deviation of an implant device size that is implanted into a patient during surgery from an implant device size defined by a surgical plan; and data indicating deviation of implant device pose after implantation into a patient during surgery from an implant device pose defined by a surgical plan.

The feedback training component 1228 may process to the post-operative feedback data to form subsets of the post-operative feedback data having similarities that satisfy a defined rule. Within each of the subsets, the feedback training component 1228 can identify correlations among at least some values of the post-operative feedback data, and then train the machine learning model based on the correlations identified for each of the subsets.

In some embodiments, the machine learning model includes a neural network component including an input layer having input nodes, a sequence of hidden layers each having a plurality of combining nodes, and an output layer having output nodes. The machine learning model is processed by at least one processing circuit (i.e., of the machine learning processing circuit 1222) configured to provide different entries of the pre-operative data to different ones of the input nodes of the neural network model, and to generate the surgical plan based on output of output nodes of the neural network component. The feedback training component 1228 may be configured to adapt weights and/or firing thresholds that are used by the combining nodes of the neural network component based on values of the post-operative feedback data.

For example, during run-time mode and training mode, the interconnected structure of the neural network between the input nodes of the input layer, the combining nodes of the hidden layers, and the output nodes of the output layer can cause the inputted values to simultaneously be processed to influence the generated output values that are used to generate the surgical plan. Each of the input nodes in the input layer multiply the input characterization data value by a weight that is assigned to the input node to generate a weighted node value. When the weighted node value exceeds a firing threshold assigned to the input node, the input node then provides the weighted node value to the combining nodes of a first one of the sequence of the hidden layers. The input node does not output the weighted node value unless if the condition is satisfied where the weighted node value exceeds the assigned firing threshold.

Furthermore, the neural network operates the combining nodes of the first one of the sequence of the hidden layers using weights that are assigned thereto to multiply and mathematically combine weighted node values provided by the input nodes to generate combined node values, and when the combined node value generated by one of the combining nodes exceeds a firing threshold assigned to the combining node to then provide the combined node value to the combining nodes of a next one of the sequence of the hidden layers. Furthermore, the neural network circuit operates the combining nodes of a last one of the sequence of hidden layers using weights that are assigned thereto to multiply and combine the combined node values provided by a plurality of combining nodes of a previous one of the sequence of hidden layers to generate combined node values, and when the combined node value generated by one of the combining nodes exceeds a firing threshold assigned to the combining node to then provide the combined node value to the output nodes of the output layer. Finally, the output nodes of the output layer is then operated to combine the combined node values from the last one of the sequences of hidden layers to generate the output values used for generating the surgical plan.

A machine learning data preconditioning circuit 1320 may be provided that pre-processes the obtained data, such as by providing normalization and/or weighting of the various types of obtained data, which is then provided to machine learning processing circuit 1222 during a run-time phase 1322 or to the feedback training component 1228 during a training phase for use in training the machine learning model 1300. In some embodiments, the training is performed continuously or at least occasionally during run-time.

The pre-operative planning component 1224 contains pre-operative data from one of the distributed network computers characterizing a defined patient, generates a surgical plan for the defined patient based on processing the pre-operative data through the machine learning model 1300, and provides the surgical plan to a display device for review by a user.

Thus, as explained above, the training can include adapting rules of an Al algorithm, rules of one or more sets of decision operations, and/or weights and/or firing thresholds of nodes of a neural network mode, to drive one or more defined key performance surgical outcomes indicated by the pre-operative data and/or the intra-operative data toward one or more defined thresholds or other rule(s) being satisfied.

The machine learning model 1300 can be configured to process the pre-operative data to output the surgical plan identifying an implant device, a pose for implantation of the implant device in the defined patient, and a predicted post-operative performance metric for the defined patient following the implantation of the implant device.

The machine learning model can be further configured to generate the surgical plan with identification of poses of resection planes for the implantation of the implant device in the defined patient. The pre-operative planning component 1224 may provide data indicating the poses of the resection planes to a computer platform 910 (e.g., Fig. 9) that generates graphical representations of the poses of the resection planes displayed though the display device within an extended reality (XR) headset 920 (Fig. 9) as an overlay on the defined patient. The pre-operative planning component 1224 may provide data indicating the poses of the resection planes to at least one controller of a surgical robot 4 (Fig. 9) to control a sequence of movements of a surgical saw attached to an arm of the surgical robot so a cutting plane of the surgical saw (e.g., surgical saw 1040 in Fig. 10 or Fig. 11) becomes sequentially aligned with the poses of the resection planes.

Is some embodiments, the machine learning model 1300 is configured to generate the surgical plan based on processing the pre-operative data comprising at least one of: joint kinematics measurement for the defined patient; soft tissue balance measurement for the defined patient; deformity correction measurement for the defined patient; and joint line measurement for the defined patient.

In some additional or alternative embodiments, the machine learning model 1300 is configured to generate the surgical plan based on processing the pre-operative data comprising at least one of: anatomical landmark locations of the defined patient; anterior reference points of the defined patient; and anatomical dimensions of the defined patient. In a further embodiment, the anatomical landmark locations identify locations of hip center, knee center, and ankle center. In a further embodiment, the anterior reference points identify a proximal tibial mechanical axis point and tibial plateau level. In a further embodiment, the anatomical dimensions identify tibial plateau size and femoral size.

During surgery (i.e., the intra-operative stage) the surgical guidance system 1220 can be configured to provide the surgical plan to a display device to assist a user (e.g., surgeon) during surgery and/or may provide the surgical plan to a robot surgery system, such as the surgical robot 4 explained above.

In some embodiments, a surgical system includes the surgical guidance system 1220 for computer assisted navigation during surgery, a tracking system, and at least one controller. As explained above, the surgical guidance system 1220 is configured to: obtain post-operative feedback data provided by distributed networked computers regarding surgical outcomes for a plurality of patients; train a machine learning model based on the post-operative feedback data; and obtain pre-operative data from one of the distributed network computers characterizing a defined patient, generate a surgical plan for the defined patient based on processing the pre-operative data through the machine learning model. The tracking system (e.g., camera tracking system component 6 and/or 6' of Fig. 9) is configured to determine a pose of an anatomical structure of the defined patient that is to be cut by a surgical saw and to determine a pose of the surgical saw. The at least one controller can be at least partially within the intra-operative guidance component 1226 and configured to obtain the surgical plan, determine a pose of a target plane based on the surgical plan defining where the anatomical structure is to be cut and based on the pose of the anatomical structure, and generate steering information based on comparison of the pose of the target plane and the pose of the surgical saw. The steering information indicates where the surgical saw needs to be moved to position a cutting plane of the surgical saw to become aligned with the target plane.

In some embodiments, the surgical system includes an XR headset 920 with at least one see-through display device. The at least one controller, which may partially reside in the computer platform 910 of Fig. 9, is configured to generate a graphical representation of the steering information that is provided to the at least one see-through display device of the XR headset 920 to guide operator movement of the surgical saw to position a cutting plane of the surgical saw to become aligned with the target plane.

In some alternative or additional embodiments, the surgical system further includes a surgical robot (e.g., surgical robot 4 above) having a robot base, a robot arm connected to the robot base and configured to position the surgical saw connected to the robot arm, and at least one motor operatively connected to move the robot arm relative to the robot base. The at least one controller is configured to control movement of the at least one motor based on the steering information to reposition the surgical saw so the cutting plane of the surgical saw becomes aligned with the target plane.

The machine learning model 1300 may be configured to process the pre-operative data to output the surgical plan identifying an implant device, poses of resection planes for the implantation of the implant device in the defined patient, and a predicted post-operative performance metric for the defined patient following the implantation of the implant device.

The surgical guidance system may be configured to train the machine learning model based on at least one of: data indicating deviation between joint kinematics measurements of the defined patient during pre-operative stage compared to during post-operative stage; data indicating deviation between tissue balance measurements of the defined patient during pre-operative stage compared to during post-operative stage; data indicating deviation between deformity correction planned for the defined patient during pre-operative stage compared to deformity correction measured for the defined patient during post-operative stage; and data indicating deviation between joint line measurements of the defined patient during pre-operative stage compared to during post-operative stage.

The surgical guidance system may be configured to train the machine learning model based on the post-operative feedback data comprising at least one of: data indicating deviation of a surgical saw cutting plane measured during surgery from a surgical saw cutting plane defined by a surgical plan; data indicating deviation of surgical saw motion measurements during surgery from surgical saw motion defined by a surgical plan; data indicating deviation of an implant device size that is implanted into a patient during surgery from an implant device size defined by a surgical plan; and data indicating deviation of implant device pose after implantation into a patient during surgery from an implant device pose defined by a surgical plan.

The machine learning model may be configured to generate the surgical plan based on processing the pre-operative data comprising at least one of: joint kinematics measurement for the defined patient; soft tissue balance measurement for the defined patient; deformity correction measurement for the defined patient; joint line measurement for the defined patient; anatomical landmark locations of the defined patient; anterior reference points of the defined patient; and anatomical dimensions of the defined patient.

The pre-operative data processed by the surgical guidance system 1220 may include at least one of: landmark locations (e.g., hip center, knee center, ankle center, etc.); femur characteristic data defining mechanical axis (hip center - distal femoral mechanical axis point, etc.), epicondylar line, Whiteside line, posterior condylar line, anterior reference point; tibia characteristic data defining mechanical axis (ankle center - proximal tibial mechanical axis point, etc.), tibial A/P direction, tibial plateau level; anatomical dimensions (e.g., tibial ML plateau size, femoral AP size, etc.); and patient demographics (e.g., age, gender, BMI, race, additions, comorbidities, etc.).

The post-operative feedback data used for training the machine learning model 1300 can include at least one of: log data structure containing listing of input data and resulting output guidance; measured outcomes (e.g., Range of Motion (ROM) test, soft tissue balance measurements, joint kinematics measurements, deformity correction measurements, joint line measurements, other functional outcomes, PROMs, patient satisfaction, etc.); surgery events (e.g., timing, problems (deviation of robot axes positions from plan, deviation of saw blade poses from plan, deviation of implant fit from predicted, unplanned user repositioning of robot arm, deviation of action tool motion from plan, unplanned surgical steps, etc.); failures (e.g., surgeon prematurely stops use of surgical robot system before plan completion, etc.); and errors (e.g., deviation of actual cutting plane from planned; deviation of predicted gap from actual gap; camera tracking system loss of tracking markers during procedure step, etc.); and observation metrics.

Inter-operative stage data that may be used for training the machine learning model 1300 can include at least one of: robot pose tracking; saw blade pose tracking; other tool and patient pose tracking; force sensor data tracking; equipment operational event tracking; and tracking camera identifiable event tracking.

The surgical plan may indicate at least one of: implant type; implant size; implant pose placement (e.g., position and rotation); predicted performance metric(s) (e.g., soft tissue balance, joint kinematics, deformity correction (e.g., hip-knee-ankle angle), joint line); resection planes (e.g., pose of each resection plane, predicted gap medial and lateral sizes, etc.); and surgery timeline.

Various embodiments herein may be used in combination with various presently available products, such as: GENU system from Globus Medical (provides pre-operative planning and intra-operative robot-assisted execution), MAKO Robotic System (Mako System) from Stryker (provides pre-operative planning and intra-operative robot-assisted execution); NAVIO (Navio System) from Smith and Nephew (provides intra-operative planning and execution); ROSA (Rosa System) from Zimmer Biomet (implements pre-operative planning, intra-operative execution assisted by a robot and post-operative follow-up using wearables and mobile application, e .g., mymobility).

Various embodiments of the present disclosure may work with one or more of the above systems or with other existing or new systems to use post-operatively obtained data for correlation with a surgical plan and execution in order to:
∘ Provide guidance information that enables a user to understand performance metrics that are predicted to be obtained through the selection of available surgical plan variables; and
∘ Provide machine learning, such may include artificial intelligence (Al), assistance to a surgeon when performing patient-specific planning:
   ▪ Defining target deformity correction(s) and/or joint line(s) through the planned surgical procedure; and/or
   ▪ Defining selection of a best implant for use with the patient.

Some other additional or alternative embodiments are directed to providing a mobile application (e.g., smartphone or other computer application) that can be communicatively connected (e.g., WiFi or Bluetooth paired) with one or more patient wearable devices for systematic data collection (functional data and Patient-reported Outcome Measures PROMs) before and after knee or other orthopedic surgery.

The machine learning processing circuit 1222 with the machine learning model 1300 can include an Al-powered algorithm component and/or neural network component that determines correlations between patient surgery outcomes, patient characteristics, surgery planning, and surgery execution.

These embodiments may be used for pre-operative planning (i.e., with or without Al Planning Assistant) with a dashboard provided through which a surgeon or other user can review previous patient performances and summary statistics of other measures present in the central database 1210. The Intra-operative guidance component 1226 can be configured for precise plan execution using robotic assistance and data collection. post-operative feedback data can include PROMs, functional and activity data collection which can be done via smart-phone application. Smart-phone application and cloud infrastructure.

Three principal imaging workflows are described which can be used with navigation system: CT-based, X-Ray based, and Imageless based workflows.

Various further operations that can be performed by the surgical guidance system 1220 will now be described in the context of Figures 14-26. Although these and other figures herein illustrate example arrows on communication paths to show a primary direction of communication, it is to be understood that communication may occur in the opposite direction to the depicted arrows. Moreover, communications can occur between other elements of these and other figures which have not been illustrated by arrows to simplify the drawings.

### Pre-Operative stage

Figure 14 illustrates functional blocks performing a pre-operative plan workflow, and which may be at least partially performed by the surgical guidance system 1220, in accordance with some embodiments. A pre-operative plan is shown in Figure 14 through which a user can plan surgery on various types of devices which may include, without limitation: desktop computer, laptop computer, mobile phone. After authentication, the user is provided new case planning or dashboard review. All planning data is synchronized with the central database. The surgery plan can be automatically synchronized across all devices, including a robot surgery system. Alternatively, the case plan can be exported to a portable storage for later import to a robot surgery system. Figure 15 illustrates functional blocks performing an example surgical case plan [WF05]. Figure 17 illustrates functional blocks for a plan device implant workflow [WF04]. The surgical case plan [WF05] can be generated by operation of the surgical guidance system 1220 and, more particularly, by the pre-operative planning component 1224.

Referring to Figure 15, a user can create a new patient case or select an existing patient case. The user can import DICOM images from various sources: CD/DVD, portable storage, hospital PACS system or central database. Later, a knee-specific application is executed which imports this case and medical image data. The user can initially select an imaging workflow for pre-operative planning, e.g.: CT imaging workflow and X-Ray imaging workflow. In next steps, the user analyzes images and plans one or more device implants. The related two workflows [WF03] for analyzing patient images is shown in Figure 15 and [WF14] for planning device(s) implant is shown in Figure 17.

There can be two variants of image analysis of the system, depending upon which imaging workflow is selected between the CT imaging and X-ray imaging, such as shown in Figure 16. Figure 16 illustrates functional blocks for image analysis and which may be at least partially performed by the surgical guidance system, in accordance with some embodiments.

For CT-based imaging workflow, the quality of CT images can be evaluated first. The quality evaluation can determine whether the CT images have sufficient quality (e.g. voxel size, coverage of hip, knee and ankle joints) and whether there was excessive patient movement during scan which may render false results. Next, CT images are segmented. In this process the 3D model of the femur and tibial bone can be generated. This process may be performed automatically without further user input, but user assistance may be provided to the process. Next important landmarks for knee planning surgery are determined: hip, knee and ankle centers, anatomical axes of femur and tibia, tibial plateau size, femoral AP size and mechanical axes.

For the X-Ray imaging workflow, the image quality and types of X-Ray images can be evaluated first. If full-leg view is available, hip, knee and ankle centers can be found and mechanical axes determined on images. If this view is not available, mechanical axis needs to be assumed based on user preferences (e.g. 6° from anatomical axes). In next steps key anatomical axes and dimensions are identified on images.

In the end, results of images analysis are shown to the user, e.g. as overlays viewed by the user on the displayed images (landmarks, axes) or 3D models (bone, CT volume rendering).

A plan device implant workflow is shown in Figure 17. First, the user selects an implant family to be used in a surgical procedure for a targeted patient. The implant family may be, for example, cruciate-retaining (CR) or posterior stabilized (PS) implants, cemented or cementless implants, etc. Next, the system can operate to automatically propose initial implant size and placement based on image analysis. In the next steps, the user can modify the implant size and placement and observe results of such modifications by updated visual feedback displayed on the medical images. When the user indicates acceptability of the plan, the plan becomes validated as approved for surgery.

A review dashboard workflow is now described. After accessing the dashboard (e.g., according to Figure 14), the machine learning processing circuit 1222 (Figure 12) selects relevant data from the central database 1010, and processes the data through the machine learning model 1300 to generate a surgical plan. The relevant data may be displayed to the user for review. Example data that can be selected for processing through the machine learning model 1300, but is not limited to any one or more of the following:
- Surgeries:
   ∘ Number of surgeries done
   ∘ Number of surgeries scheduled
- Timing analysis:
   ∘ Average
   ∘ Shortest
   ∘ Longest
- Precision analysis (planned vs. done): Average
- Patient demographics:
   ∘ Age
   ∘ Gender
   ∘ BMI
   ∘ Race
   ∘ Additions
   ∘ Comorbidities
- Implant statistics:
   ∘ Types
   ∘ Sizes
- Outcomes:
   ∘ PROMs
   ∘ Functional outcomes
   ∘ Patient satisfaction
- Surgery events: Problems, failures, errors, observations
- Analysis:
   ∘ Outcomes vs precision
   ∘ Outcomes vs. timing
   ∘ Outcomes vs. patient demographics
   ∘ Outcomes vs. implants
   ∘ Outcomes vs. surgery events

The data processed by the machine learning processing circuit 1222 and which may be may be displayed to the user (e.g., surgeon) may, example, help a surgeon to track performance over time using dashboard, and/or enable the surgeon to improve performance by reviewing easily accessible analysis data from, e.g., the surgeon's own surgeries, surgeries performed by other surgeons (which may be anonymized), and/or reviewing whole episode of

### Intra-operative Workflows

Intra-operative workflow may begin with system set-up. Nurse brings robot and navigation station to the OR and connects to mains. System uses standard power mains outlet. After booting the system asks for authentication. If successful, the user can import previously planned case from portable storage or from the central database 1210 (e.g., workflow in Figure 14). User has the possibility to fully plan the case, including image analysis, intra-operatively. Before continuing with the case, the surgeon will have to confirm case-relevant data on a screen showing summary of planning.

In parallel to case configuration and patient plan, nurses can drape the robot and attach End Effector Arm (EEA) to robot flange. Navigated instruments are assembled next. They are verified using divots on Dynamic Reference Arrays (DRA) or EEA Reference Element (RE). Saw blade is attached to EEA and saw handpiece attached to saw blade. Last, EEA and saw blade are verified by navigation system using divot on saw blade.

Patient registration workflow includes having the patient installed on an OR table with leg in a leg holder (standard leg holder from "De Mayo V2 Surgical Positioning hip center holder" from IMP). Next surgeon makes a standard opening for TKA. Femur and tibial DRAs are installed using bone pins. Surveillance markers are installed in femur and tibia. User can adjust camera position to ensure that all the REs are correctly visible.

As a first part of the registration process, hip center of rotation is found. It is achieved by surgeon making rotational movements of the leg while navigation registers femur positions. Surgeon shall be assisted by relevant indications on the computer screen about range of motion necessary for hip center registration. As hip is principally a spherical joint, it's center can be calculated from series of femur DRA position measurements.

Next natural landmarks on femur and tibia are measured using navigated stylus. These are for femur: mechanical axis (hip center - distal femoral mechanical axis point), epicondylar line, Whiteside line, posterior condylar line, anterior reference point and for tibia: mechanical axis (ankle center - proximal tibial mechanical axis point), tibial A/P direction, tibial plateau level. Software assists user in collecting these points.

Depending on selected imaging workflow, there are the following ways to register distal end of the femur, as follows:

(1) For CT-based workflow an optional coarse femur registration step is done first. In this step, user will measure pre-planned points on the surface of the bone. It is possible that this step of collecting pre-planned points will not be required and natural landmarks measured in previous step will be enough. Next user will collect several (10 to 50) points on the femur bone surface. This might require using sharp stylus to be able to reach the bone through cartilage. Software might assist user in this task by showing him areas that have not been covered by points. Automatic surface matching algorithm (e.g. Iterative Closes Point (ICP) algorithm) matches measured points with segmented bone model and calculates matching error. If error is sufficiently small, registration matrix is stored, and femur registered. Similar process is used to register tibia.

(2) For X-ray and imageless workflows, only condylar surfaces are measured in addition to previously collected natural landmarks. This is to complete the virtual model of the patient obtained intra-operatively called "stickman" which is composed of natural landmarks and knee joint surfaces.

Next, surgeon measures divots on surveillance markers using stylus to define which surveillance marker is linked to which DRA (femoral or tibial). In the end surgeon will review registration precision by touching selected points on bone with a stylus and verifying that on the navigation screen pointer tip is placed on the bone. If registration precision is sufficiently high, surgeon will move to the next step.

In some embodiments the registration is performed using fluoroscopy-matching and/or surface scanning using a laser scanner.

After patient is registered, navigation information is displayed, which may include showing in real time tibia and femur bone positions together with summary of important information for review by the surgeon. Examples of this information include: instantaneous varus / valgus, knee flexion / extension, internal / external rotation angles, gap size, using, for example:
∘ Femoral and tibial markers
∘ Virtual model ("stickman")
∘ Registration transform
∘ Planned implants

The displayed information may include a 3D model that is generated or obtained.

Surgeon can move the leg across range of motion while observing the values in order to learn more about patient joint structure, kinematics and soft tissue balance.

Data for the navigation display come from the navigation workflow shown in Figure 18. Figure 18 illustrates functional blocks for navigated workflow and which may be at least partially performed by the surgical guidance system (e.g. the intra-operative guidance component 1226) in accordance with some embodiments. The data may be calculated in real time based on DRAs measurements by the tracking system and robot and EEA position measurements by integrated encoders. Using transformation matrices and robot models, positions of bones, instrument, robotic arm links and EEA can be calculated and used across the application. Additionally, surveillance markers are tracked in real time in reference to their respective DRAs. If the relative position between the two changes too much, it indicates that possibly a DRA has moved in reference to bone and registration lost. In such case user will be warned and registration will have to be redone.

In some embodiments, navigation data indicating the angles and measurements could be displayed through an XR headset an overlay directly on the patient anatomy.

In order to obtain information about soft tissue balance and joint kinematics, surgeon performs Range of Motion ROM test, such as shown in Figure 19. Figure 19 illustrates functional blocks for testing Range of Motion (ROM) workflow and which may be at least partially performed by the surgical guidance system in accordance with some embodiments. In this workflow, a user can move the leg across full ROM define number of times, e.g. three times: without applying any lateral forces, applying varus force and applying valgus force. The surgical guidance system, e.g., the pre-operative planning component 1224 or other component of a navigation system, will collect relative positions of femur and tibia and calculate maximal gap size for lateral and medical compartment and maximal varus/valgus angle in function of flexion angle, which will be used in the implant planning stage.

In some embodiments, a robot arm can be attached to the leg and moved along paths to generate standardized ROM movement while collecting reaction (e.g., tracked poses of the robot arm and/or sense force feedback) from the leg to establish objective and repeatable reference across surgeons and patients.

During implant planning (as shown in Figure 17), the surgeon or other user can change position and rotation of the implant in reference to bone while observing in real time its impact on ligaments balancing. This can be achieved by showing a graph with original and planned medial and lateral gap sizes across acquired range of motion. The planned graph will be updated when the implant size and position is modified. Additionally, the system will assist surgeon in intra-op planning by providing ligament balancing information at current flexion angle and display of gap medial and lateral sizes at currently measured flexion angle. This data will be updated when the implant size and position, or the flexion angle is modified.

The surgical guidance system can operate to assist the user in intra-op implant planning by providing features which may include any one or more of the following:
- For CT-workflow, 3D overlay views of tibia and femur with:
   ∘ 3D model
   ∘ Planned implant
   ∘ Resection planes
   ∘ CT slice at selectable view depth (section view)
- For image-less / X-ray workflow, overlay views of tibia and femur composed of generic 3D model and planned implant.
- All: display natural landmarks and associated "stickman".

As a last stage of planning, the surgeon can validate the displayed plan.

The surgical plan can be displayed through an XR headset or other display used in implant planning stage. Surgeon could see a virtual overlay of the implant directly on patient bone and be able to interact with it using handles for example to rotate or translate it in reference to the bone, such as shown in Figure 20. Figure 20 illustrates part of a surgical plan is displayed through an XR headset as an overlay on a patient's bone to assist with implant position planning in accordance with some embodiments.

Figure 21 shows a surgical robot workflow performed to make one or more cuts on a bone according to a surgical plan, and according to some embodiments. In order to cut bones with robotic assistance, robot station needs to be brought to the table. The system guides user in positioning the system by showing when all planned cutting planes are within robot workspace without need to move the robot station. Next, robot station is stabilized and optionally the saw blade is one more time verified using a divot.

Surgeon selects resection plane, such as proximal for tibia, distal, anterior, anterior chamfer, posterior or posterior chamfer for femur. System ensures that the registration is valid either automatically if surveillance markers are visible of requiring user to measure surveillance marker divot. Robotic arm moves to the target position where the resection plane is in EEA workspace and aligned with EEA cutting plane. Surgeon unlocks the lock and drives the saw until the bone is removed. During this stage, the system shows on the navigation screen the position of the saw blade in reference to bone as well as remaining bone to remove.

Figure 22 shows a check planarity workflow which may be at least partially performed by the surgical guidance system in accordance with some embodiments. After each cut surgeon may measure planarity of the cut by placing plane checker on the bone and verifying on the screen deviation of the executed plane from the planned plane.

Alternatively, if robotic system is not to be used, e.g. due to failure, the surgeon still has the possibility to cut bones manually with navigation assistance. Similarly to robotic cutting, surgeon selects resection plane and registration is verified. Then there are two possibilities depending on target implementation:
- Navigated jigs: system indicates to surgeon which navigated jig to use and shows target position. Surgeon fixes the jig to bone in location indicated by the system (e.g. system indicates target position in green if navigated jigs is within certain maximal distance from it) and cuts manually using sagittal saw through the jig.
- Navigated pin guide: system guides user in placing pins in bone. Later user slides appropriate jig on pins and cuts manually.

Planarity of the cut can be verified.

Figure 23 shows a workflow to cut bones with navigated jigs which may be at least partially performed by the surgical guidance system in accordance with some embodiments.

After doing cuts all cuts, surgeon places trial implants and can test ROM as described before. If he is not satisfied with soft tissue balancing, he can re-plan implant and redo the cuts. Afterwards, the surgeon may place final implants with or without cement.

Upon reaching the end of the surgery, surgeon will evaluate results. This is done by measuring implants positions using stylus and divots in implants such as shown in Figures 24 and 25. Figure 24 shows a workflow to evaluate results of implantation of the implant device, which may be at least partially performed by the surgical guidance system, in accordance with some embodiments. Figure 25 shows divots provided on implant devices which can have their collective poses tracked by the camera tracking system in accordance with some embodiments. By proper spatial disposition and number of divots implant type, position and size shall be identified. Surgery summary will be displayed to the surgeon:
- Measured implant positions
- Implant type and size
- Last ROM test results
- Surgery timeline

The system will store key surgery data and upload it to the central database 1210 for use in training of the machine learning model 1300 by the feedback training component 1228:
- System and software versions
- Case identification
- UI telemetry
- State machine:
   ∘ Timestamps
   ∘ Signals
   ∘ States
- Registration data:
   ∘ Measured natural landmarks (points, surfaces and relevant tracking errors)
   ∘ Registration transformations
   ∘ Registration precision (CT imaging workflow)
- Setup:
   ∘ Position of reference elements and DRAs in space
   ∘ Verification measurements (divots on navigated instruments)
- Planning:
   ∘ References to any medical images used
   ∘ Segmentation
   ∘ Planned implant positions
- Robot station and EEA:
   ∘ Robot axes positions while moving (up to 20 Hz)
   ∘ EEA and saw blade positions when unlocked (up to 20 Hz)
   ∘ Aggregated force sensor data
- ROM test:
   ∘ Source data and results for each completed ROM test
- Final evaluation of implant components (tibial, femoral, PE):
   ∘ Divot measurements
   ∘ Position
   ∘ Size
   ∘ Type
- Tracking:
   ∘ Aggregated positions and errors of navigated instruments and markers
   ∘ Any tracking issues

### Post-operative stage

Figure 26 shows a patient examination workflow which may be at least partially performed by the surgical guidance system, in accordance with some embodiments.

In the post-operative stage, the surgical guidance system is configured to collect relevant result data and enable in-house patient rehabilitation. It has been demonstrated that professional rehabilitation after TKA does not have better outcomes than auto-rehabilitation done by the patient. On the other hand, the patient needs to be accompanied after discharge and know what type of exercise he shall do. Some embodiments provide a mobile application which can be installed on a mobile phone or tablet. Mobile application can be configured to periodically query the patient to assess the patient's status via Patient Reported Outcome Measures PROMs questionnaires. A wearable device linked with the mobile device (e.g. smartwatch, shoe sensor) will measure patient activity. On the mobile application there will be a customizable rehabilitation program including instructional videos and a way to check if patient is following defined rehabilitation processes (e.g. via follow-up questions and wearable device signal processing). In case of emergency, the patient can contact hospital via the application for immediate advice.

All patient data will be collected, uploaded to the central database 1210 for storage and linked with particular patient and case. Hospital staff and surgeon will have real-time view of all patients enrolled in the program with highlights on case outside standard workflow (e.g. very low activity, bad PROMs). Hospital staff and surgeon shall be able to contact patient if they need. The feedback training component 1228 can use this post-surgical outcome data to further train the machine learning model 1300.

The central database 1210 is used to collect the outcome data and link them with the data from whole episode of care, e.g., as shown in Figure 12. The machine learning model 1300 can be trained over time based on the collected data to identify the best treatment options, including implant type, dimensions, and placement, target deformity correction, target joint line for a particular patient. As explained above, the machine learning model 1300 may use artificial intelligence algorithms to find correlations and used practically in the implant planning stage by providing Al planning assistant and surgeon dashboard extension.

### Further Definitions and Embodiments:

In the above-description of various embodiments of present inventive concepts, it is to be understood that the terminology used herein is for the purpose of describing particular embodiments only and is not intended to be limiting of present inventive concepts. Unless otherwise defined, all terms (including technical and scientific terms) used herein have the same meaning as commonly understood by one of ordinary skill in the art to which present inventive concepts belongs. It will be further understood that terms, such as those defined in commonly used dictionaries, should be interpreted as having a meaning that is consistent with their meaning in the context of this specification and the relevant art and will not be interpreted in an idealized or overly formal sense expressly so defined herein.

When an element is referred to as being "connected", "coupled", "responsive", or variants thereof to another element, it can be directly connected, coupled, or responsive to the other element or intervening elements may be present. In contrast, when an element is referred to as being "directly connected", "directly coupled", "directly responsive", or variants thereof to another element, there are no intervening elements present. Like numbers refer to like elements throughout. Furthermore, "coupled", "connected", "responsive", or variants thereof as used herein may include wirelessly coupled, connected, or responsive. As used herein, the singular forms "a", "an" and "the" are intended to include the plural forms as well, unless the context clearly indicates otherwise. Well-known functions or constructions may not be described in detail for brevity and/or clarity. The term "and/or" includes any and all combinations of one or more of the associated listed items.

It will be understood that although the terms first, second, third, etc. may be used herein to describe various elements/operations, these elements/operations should not be limited by these terms. These terms are only used to distinguish one element/operation from another element/operation. Thus, a first element/operation in some embodiments could be termed a second element/operation in other embodiments without departing from the teachings of present inventive concepts. The same reference numerals or the same reference designators denote the same or similar elements throughout the specification.

As used herein, the terms "comprise", "comprising", "comprises", "include", "including", "includes", "have", "has", "having", or variants thereof are open-ended, and include one or more stated features, integers, elements, steps, components or functions but does not preclude the presence or addition of one or more other features, integers, elements, steps, components, functions or groups thereof. Furthermore, as used herein, the common abbreviation "e.g.", which derives from the Latin phrase "exempli gratia," may be used to introduce or specify a general example or examples of a previously mentioned item, and is not intended to be limiting of such item. The common abbreviation "i.e.", which derives from the Latin phrase "id est," may be used to specify a particular item from a more general recitation.

Example embodiments are described herein with reference to block diagrams and/or flowchart illustrations of computer-implemented methods, apparatus (systems and/or devices) and/or computer program products. It is understood that a block of the block diagrams and/or flowchart illustrations, and combinations of blocks in the block diagrams and/or flowchart illustrations, can be implemented by computer program instructions that are performed by one or more computer circuits. These computer program instructions may be provided to a processor circuit of a general purpose computer circuit, special purpose computer circuit, and/or other programmable data processing circuit to produce a machine, such that the instructions, which execute via the processor of the computer and/or other programmable data processing apparatus, transform and control transistors, values stored in memory locations, and other hardware components within such circuitry to implement the functions/acts specified in the block diagrams and/or flowchart block or blocks, and thereby create means (functionality) and/or structure for implementing the functions/acts specified in the block diagrams and/or flowchart block(s).

These computer program instructions may also be stored in a tangible computer-readable medium that can direct a computer or other programmable data processing apparatus to function in a particular manner, such that the instructions stored in the computer-readable medium produce an article of manufacture including instructions which implement the functions/acts specified in the block diagrams and/or flowchart block or blocks. Accordingly, embodiments of present inventive concepts may be embodied in hardware and/or in software (including firmware, resident software, micro-code, etc.) that runs on a processor such as a digital signal processor, which may collectively be referred to as "circuitry," "a module" or variants thereof.

It should also be noted that in some alternate implementations, the functions/acts noted in the blocks may occur out of the order noted in the flowcharts. For example, two blocks shown in succession may in fact be executed substantially concurrently or the blocks may sometimes be executed in the reverse order, depending upon the functionality/acts involved. Moreover, the functionality of a given block of the flowcharts and/or block diagrams may be separated into multiple blocks and/or the functionality of two or more blocks of the flowcharts and/or block diagrams may be at least partially integrated. Finally, other blocks may be added/inserted between the blocks that are illustrated, and/or blocks/operations may be omitted without departing from the scope of inventive concepts. Moreover, although some of the diagrams include arrows on communication paths to show a primary direction of communication, it is to be understood that communication may occur in the opposite direction to the depicted arrows.

Many variations and modifications can be made to the embodiments without substantially departing from the principles of the present inventive concepts. All such variations and modifications are intended to be included herein within the scope of present inventive concepts. Accordingly, the above disclosed subject matter is to be considered illustrative, and not restrictive, and the appended examples of embodiments are intended to cover all such modifications, enhancements, and other embodiments, which fall within the spirit and scope of present inventive concepts. Thus, to the maximum extent allowed by law, the scope of present inventive concepts are to be determined by the broadest permissible interpretation of the present disclosure including the following examples of embodiments and their equivalents, and shall not be restricted or limited by the foregoing detailed description.

The invention could be defined inter alai by the following examples:
1. A surgical guidance system for computer assisted navigation during surgery, the surgical guidance system configured to: obtain post-operative feedback data provided by distributed networked computers regarding surgical outcomes for a plurality of patients train a machine learning model based on the post-operative feedback data; obtain pre-operative data from one of the distributed network computers characterizing a defined patient; generate a surgical plan for the defined patient based on processing the pre-operative data through the machine learning model; and provide the surgical plan to a display device for review by a use
2. The surgical guidance system of Example 1, wherein the machine learning model is configured to: process the pre-operative data to output the surgical plan identifying an implant device, a pose for implantation of the implant device in the defined patient, and a predicted post-operative performance metric for the defined patient following the implantation of the implant device
3. The surgical guidance system of Example 2, wherein the machine learning model is further configured to: generate the surgical plan with identification of poses of resection planes for the implantation of the implant device in the defined patient.
4. The surgical guidance system of Example 3 further configured to: provide data indicating the poses of the resection planes to a computer platform that generates graphical representations of the poses of the resection planes displayed though the display device within an extended reality (XR) headset as an overlay on the defined patient.
5. The surgical guidance system of Example 3 further configured to: provide data indicating the poses of the resection planes to at least one controller of a surgical robot to control a sequence of movements of a surgical saw attached to an arm of the surgical robot so a cutting plane of the surgical saw becomes sequentially aligned with the poses of the resection planes.
6. The surgical guidance system of Example 1 further configured to train the machine learning model based on the post-operative feedback data comprising at least one of: joint kinematics measurements; soft tissue balance measurements; deformity correction measurements; joint line measurements; and patient reported outcome measures.
7. The surgical guidance system of Example 1 further configured to train the machine learning model based on at least one of: data indicating deviation between joint kinematics measurements of the defined patient during pre-operative stage compared to during post-operative stage; data indicating deviation between tissue balance measurements of the defined patient during pre-operative stage compared to during post-operative stage; data indicating deviation between deformity correction planned for the defined patient during pre-operative stage compared to deformity correction measured for the defined patient during post-operative stage; and data indicating deviation between joint line measurements of the defined patient during pre-operative stage compared to during post-operative stage.
8. The surgical guidance system of Example 1 further configured to train the machine learning model based on the post-operative feedback data comprising at least one of: data indicating deviation of a surgical saw cutting plane measured during surgery from a surgical saw cutting plane defined by a surgical plan; data indicating deviation of surgical saw motion measurements during surgery from surgical saw motion defined by a surgical plan; data indicating deviation of an implant device size that is implanted into a patient during surgery from an implant device size defined by a surgical plan; and data indicating deviation of implant device pose after implantation into a patient during surgery from an implant device pose defined by a surgical plan
9. The surgical guidance system of Example 1 further configured to: form subsets of the post-operative feedback data having similarities that satisfy a defined rule; within each of the subsets, identify correlations among at least some values of the post-operative feedback data; and train the machine learning model based on the correlations identified for each of the subsets.
10. The surgical guidance system of Example 1, wherein the machine learning model comprises: a neural network component including an input layer having input nodes, a sequence of hidden layers each having a plurality of combining nodes, and an output layer having output nodes; and at least one processing circuit configured to provide different entries of the pre-operative data to different ones of the input nodes of the neural network model, and to generate the surgical plan based on output of output nodes of the neural network component.
11. The surgical guidance system of Example 10, further comprising a feedback training component configured to: adapt weights and/or firing thresholds that are used by the combining nodes of the neural network component based on values of the post-operative feedback data.
12. The surgical guidance system of Example 1, wherein the machine learning model is configured to generate the surgical plan based on processing the pre-operative data comprising at least one of: joint kinematics measurement for the defined patient; soft tissue balance measurement for the defined patient; deformity correction measurement for the defined patient; and joint line measurement for the defined patient.
13. The surgical guidance system of Example 1, wherein the machine learning model is configured to generate the surgical plan based on processing the pre-operative data comprising at least one of: anatomical landmark locations of the defined patient; anterior reference points of the defined patient; and anatomical dimensions of the defined patient.
14. The surgical guidance system of Example 13, wherein: the anatomical landmark locations identify locations of hip center, knee center, and ankle center; the anterior reference points identify a proximal tibial mechanical axis point and tibial plateau level; and the anatomical dimensions identify tibial plateau size and femoral size.
15. A surgical system comprising: a surgical guidance system for computer assisted navigation during surgery, the surgical guidance system configured to, obtain post-operative feedback data provided by distributed networked computers regarding surgical outcomes for a plurality of patients, train a machine learning model based on the post-operative feedback data, and obtain pre-operative data from one of the distributed network computers characterizing a defined patient, generate a surgical plan for the defined patient based on processing the pre-operative data through the machine learning model; a tracking system configured to determine a pose of an anatomical structure of the defined patient that is to be cut by a surgical saw and to determine a pose of the surgical saw; and at least one controller configured to obtain the surgical plan from the surgical guidance system, determine a pose of a target plane based on the surgical plan defining where the anatomical structure is to be cut and based on the pose of the anatomical structure, and generate steering information based on comparison of the pose of the target plane and the pose of the surgical saw, wherein the steering information indicates where the surgical saw needs to be moved to position a cutting plane of the surgical saw to become aligned with the target plane.
16. The surgical system of Example 15, further comprising: an extended reality (XR) headset including at least one see-through display device, wherein the at least one controller is configured to generate a graphical representation of the steering information that is provided to the at least one see-through display device of the XR headset to guide operator movement of the surgical saw to position a cutting plane of the surgical saw to become aligned with the target plane.
17. The surgical system of Example 15, further comprising: a surgical robot including, a robot base, a robot arm connected to the robot base and configured to position the surgical saw connected to the robot arm, and at least one motor operatively connected to move the robot arm relative to the robot base, wherein the at least one controller is configured to control movement of the at least one motor based on the steering information to reposition the surgical saw so the cutting plane of the surgical saw becomes aligned with the target plane.
18. The surgical system of Example 15, wherein the machine learning model is configured to: process the pre-operative data to output the surgical plan identifying an implant device, poses of resection planes for the implantation of the implant device in the defined patient, and a predicted post-operative performance metric for the defined patient following the implantation of the implant device.
19. The surgical system of Example 15, wherein the surgical guidance system is configured to train the machine learning model based on at least one of: data indicating deviation between joint kinematics measurements of the defined patient during pre-operative stage compared to during post-operative stage; data indicating deviation between tissue balance measurements of the defined patient during pre-operative stage compared to during post-operative stage; data indicating deviation between deformity correction planned for the defined patient during pre-operative stage compared to deformity correction measured for the defined patient during post-operative stage; and data indicating deviation between joint line measurements of the defined patient during pre-operative stage compared to during post-operative stage.
20. The surgical system of Example 15, wherein the surgical guidance system is configured to train the machine learning model based on the post-operative feedback data comprising at least one of: data indicating deviation of a surgical saw cutting plane measured during surgery from a surgical saw cutting plane defined by a surgical plan; data indicating deviation of surgical saw motion measurements during surgery from surgical saw motion defined by a surgical plan; data indicating deviation of an implant device size that is implanted into a patient during surgery from an implant device size defined by a surgical plan; and data indicating deviation of implant device pose after implantation into a patient during surgery from an implant device pose defined by a surgical plan.
21. The surgical system of Example 15, wherein the machine learning model is configured to generate the surgical plan based on processing the pre-operative data comprising at least one of: joint kinematics measurement for the defined patient; soft tissue balance measurement for the defined patient; deformity correction measurement for the defined patient; joint line measurement for the defined patient; anatomical landmark locations of the defined patient; anterior reference points of the defined patient; and anatomical dimensions of the defined patient.

## Claims

1. A surgical guidance system for computer assisted navigation during surgery, the surgical guidance system configured to:
- obtain post-operative feedback data provided by distributed networked computers regarding surgical outcomes for a plurality of patients;
- train a machine learning model based on the post-operative feedback data;
- obtain pre-operative data from one of the distributed network computers characterizing a defined patient;
- generate a surgical plan for the defined patient based on processing the pre-operative data through the machine learning model; and
- provide the surgical plan to a display device for review by a user.

2. The surgical guidance system of Claim 1, wherein the machine learning model is configured to: process the pre-operative data to output the surgical plan identifying an implant device, a pose for implantation of the implant device in the defined patient, and a predicted post-operative performance metric for the defined patient following the implantation of the implant device.

3. The surgical guidance system of Claim 2, wherein the machine learning model is further configured to: generate the surgical plan with identification of poses of resection planes for the implantation of the implant device in the defined patient and it is preferably further configured to: provide data indicating the poses of the resection planes to a computer platform that generates graphical representations of the poses of the resection planes displayed though the display device within an extended reality (XR) headset as an overlay on the defined patient.

4. The surgical guidance system of Claim 3 further configured to: provide data indicating the poses of the resection planes to at least one controller of a surgical robot to control a sequence of movements of a surgical saw attached to an arm of the surgical robot so a cutting plane of the surgical saw becomes sequentially aligned with the poses of the resection planes.

5. The surgical guidance system of Claim 1 further configured to train the machine learning model based on the post-operative feedback data comprising at least one of:
- joint kinematics measurements;
- soft tissue balance measurements;
- deformity correction measurements;
- joint line measurements; and
- patient reported outcome measures.

6. The surgical guidance system of Claim 1 further configured to train the machine learning model based on at least one of:
- data indicating deviation between joint kinematics measurements of the defined patient during pre-operative stage compared to during post-operative stage;
- data indicating deviation between tissue balance measurements of the defined patient during pre-operative stage compared to during post-operative stage;
- data indicating deviation between deformity correction planned for the defined patient during pre-operative stage compared to deformity correction measured for the defined patient during post-operative stage; and
- data indicating deviation between joint line measurements of the defined patient during pre-operative stage compared to during post-operative stage.

7. The surgical guidance system of Claim 1 further configured to train the machine learning model based on the post-operative feedback data comprising at least one of:
- data indicating deviation of a surgical saw cutting plane measured during surgery from a surgical saw cutting plane defined by a surgical plan;
- data indicating deviation of surgical saw motion measurements during surgery from surgical saw motion defined by a surgical plan;
- data indicating deviation of an implant device size that is implanted into a patient during surgery from an implant device size defined by a surgical plan; and
- data indicating deviation of implant device pose after implantation into a patient during surgery from an implant device pose defined by a surgical plan.

8. The surgical guidance system of Claim 1 further configured to:
- form subsets of the post-operative feedback data having similarities that satisfy a defined rule;
- within each of the subsets, identify correlations among at least some values of the post-operative feedback data; and
- train the machine learning model based on the correlations identified for each of the subsets.

9. The surgical guidance system of Claim 1, wherein the machine learning model comprises:
- a neural network component including an input layer having input nodes, a sequence of hidden layers each having a plurality of combining nodes, and an output layer having output nodes; and
- at least one processing circuit configured to provide different entries of the pre-operative data to different ones of the input nodes of the neural network model, and to generate the surgical plan based on output of output nodes of the neural network component.

10. The surgical guidance system of Claim 10, further comprising a feedback training component configured to: adapt weights and/or firing thresholds that are used by the combining nodes of the neural network component based on values of the post-operative feedback data.

11. The surgical guidance system of Claim 1, wherein the machine learning model is configured to generate the surgical plan based on processing the pre-operative data comprising at least one of:
- joint kinematics measurement for the defined patient;
- soft tissue balance measurement for the defined patient;
- deformity correction measurement for the defined patient; and
- joint line measurement for the defined patient.

12. The surgical guidance system of Claim 1, wherein the machine learning model is configured to generate the surgical plan based on processing the pre-operative data comprising at least one of:
- anatomical landmark locations of the defined patient;
- anterior reference points of the defined patient; and
anatomical dimensions of the defined patient, and preferably wherein .
- the anatomical landmark locations identify locations of hip center, knee center, and ankle center;
- the anterior reference points identify a proximal tibial mechanical axis point and tibial plateau level; and
- the anatomical dimensions identify tibial plateau size and femoral size.

13. A surgical system comprising:
- a surgical guidance system for computer assisted navigation during surgery according to any one of the preceding claims;
- a tracking system configured to determine a pose of an anatomical structure of the defined patient that is to be cut by a surgical saw and to determine a pose of the surgical saw; and
- at least one controller configured to obtain the surgical plan from the surgical guidance system, determine a pose of a target plane based on the surgical plan defining where the anatomical structure is to be cut and based on the pose of the anatomical structure, and generate steering information based on comparison of the pose of the target plane and the pose of the surgical saw, wherein the steering information indicates where the surgical saw needs to be moved to position a cutting plane of the surgical saw to become aligned with the target plane.

14. The surgical system of Claim 13, further comprising:
- an extended reality (XR) headset including at least one see-through display device,
- wherein the at least one controller is configured to generate a graphical representation of the steering information that is provided to the at least one see-through display device of the XR headset to guide operator movement of the surgical saw to position a cutting plane of the surgical saw to become aligned with the target plane.

15. The surgical system of Claim 14, further comprising: a surgical robot including,
- a robot base,
- a robot arm connected to the robot base and configured to position the surgical saw connected to the robot arm, and
- at least one motor operatively connected to move the robot arm relative to the robot base,
- wherein the at least one controller is configured to control movement of the at least one motor based on the steering information to reposition the surgical saw so the cutting plane of the surgical saw becomes aligned with the target plane.
